# EUROPÄISCHE PATENTSCHRIFT

(11) **EP 0 446 173 B1**
(45) Veröffentlichungstag und Bekanntmachung des Hinweises auf die Patenterteilung: **14.07.1999**
(21) Anmeldenummer: 91810132.0
(22) Anmeldetag: 28.02.1991
(51) Int. Cl.: C12N 5/20, C12P 21/08, G01N 33/577, G01N 33/543, C07C 233/25, C07C 323/12

(54) **Immunologischer Nachweis von Metolachlor**
Immunoassay for metolachlorine
Essai immunologique du métolachlore

(30) Priorität: 09.03.1990 CH 76290; 23.10.1990 CH 337890
(43) Veröffentlichungstag der Anmeldung: 11.09.1991
(73) Patentinhaber: Novartis AG, 4058 Basel (CH); Novartis-Erfindungen Verwaltungsgesellschaft m.b.H., 1235 Wien (AT)
(72) Erfinder: Schlaeppi, Jean-Marc, Dr., CH-4051 Basel (CH); Ramsteiner, Klaus, Dr., CH-4114 Hofstetten (CH); Moser, Hans, Dr., CH-4312 Magden (CH)

(56) Entgegenhaltungen:
- EP-A- 340 198
- AGRIC.FOOD CHEM., Bd. 40, 1992, Seiten 211 - 214 FENG ET AL. 'A GENERAL METHOD FOR DEVELOPING IMMUNOASSAYS TO CHLOROACETANILIDE HERBICIDES'

## Beschreibung

Gegenstand der vorliegenden Erfindung sind monoklonale Antikörper, die sich durch eine hohe Selektivität und Affinität gegenüber Metolachlor auszeichnen und die sich daher in hervorragender Weise für die Verwendung in einem Immunassay zum schnellen und effektiven Nachweis von Metolachlor eignen.

Ein weiterer Aspekt der vorliegenden Erfindung betrifft Hybridomazellinien, die besagte monoklonale Antikörper produzieren sowie immunologische Verfahren zum Nachweis von Metolachlor unter Verwendung besagter monoklonaler Antikörper und die im Rahmen dieser Nachweisverfahren verwendbaren Testkits.

Der Einsatz synthetischer Herbizide für die Zwecke des Pflanzenschutzes und die damit in Zusammenhang stehenden Umweltbelastungen sind in jüngster Zeit immer häufiger in den Mittelpunkt der öffentlichen Diskussion gerückt.

Metolachlor ist ein weitverbreitetes Herbizid, das für die Zwecke des Pflanzenschutzes eingesetzt wird, insbesondere zur selektiven Bekämpfung verschiedener Wildgräser in Mais-, Baumwoll-, Kartoffel-, Sorghum- und Zuckerrübenkulturen.

Chemisch gesehen handelt es sich beim Metolachlor um 2-Chlor-6'-ethyl-N-(2-methoxy-1-methylethyl)acet-O-toluidid [siehe: The Pesticide Manual, 8th Edition, ed. by C.R. Worthing and S.B. Walker, The British Crop Protection Council, Thornton Heath CR4 7QG, UK, 1987, Seiten 568 und 569].

Der Nachweis von Metolachlor in Boden- und Wasserproben wird heute in erster Linie mittels Gas- oder Flüssigchromatographie (z.B. HPLC) durchgeführt [Hargrave HS und Merkle MG (1971); van Rensburg E (1985)].
All die genannten Verfahren zum Nachweis synthetischer Herbizide sind jedoch mit zahlreichen Nachteilen verbunden. So müssen beispielsweise für die Bestimmung von Metolachlor in Bodenproben mittels GC oder HPLC vor der Durchführung der eigentlichen chromatographischen Analyse aufwendige Reinigungs- und Aufkonzentrierungsschritte zwischengeschaltet werden.

Weitere Nachteile dieser Verfahren sind darin zu sehen, dass beispielsweise in der Gas-chromatographie elementspezifische Detektoren eingesetzt werden, während bei der HPLC photometrische Detektoren verwendet werden, die relativ unspezifisch sind. Mit Ausmahme der massenspektroskopischen Detektion beruhen die chromatographischen Analysen auf der Bestimmung von Retentionszeiten der jeweiligen Substanz. Diese Werte sind aber relativ und damit nicht strukturspezifisch.

Um diese zuvor beschriebenen Nachteile der etablierten analytischen Verfahren zu vermeiden, hat man in jüngster Zeit versucht auch für den Agrarsektor immunologische Verfahren zu entwickeln, - wie sie in der klinischen Diagnostik zum Nachweis der verschiedensten Antigene bereits routinemässig eingesetzt werden- , insbesondere für die quantitative und qualitative Bestimmung von Agrarchemikalien in Boden-, Wasser- oder Luftproben.

So hat man beispielsweise mittlerweile begonnen immunologische Verfahren für den Nachweis bestimmter Herbizide, wie 2,4-Dichlorphenoxyessigsäure (Fleeker, 1986) oder Chlorsulfuron (Kelley *et al*, 1985) sowie verschiedener Pestizide, wie Diflubenzuron (Wie und Hammock, 1982), Metalaxyl (Newsome, 1985), Alachlor (Feng *et al*, 1990) oder Parathion (Ercegovich *et al*, 1981) zu entwickeln.
Auch für den immunologischen Nachweis von Atrazin ist bereits ein Verfahren beschrieben (US-P 4,530,786), das jedoch, wie auch die zuvor genannten Methoden, auf der Verwendung polyklonaler Antiseren beruht, die aus Tieren gewonnen werden, welche zuvor mit einem entsprechenden Antigen immunisiert wurden.

Polyklonale Antiseren sind in ihrer Zusammensetzung sehr heterogen, d.h. sie enthalten eine Vielzahl verschiedener Antikörper, die mit unterschiedlichen Epitopen des jeweiligen Antigens reagieren. Diese heterogene Zusammensetzung polyklonaler Antiseren kommt dadurch zustande, dass bei der Immunisierung eines Versuchstieres mit einem bestimmten Antigen immer mehrere Antikörper-produzierende Zellklone gleichzeitig stimuliert werden, von denen jeder ein anderes Epitop auf dem Antigenmolekül erkennt und daher von den stimulierten Zellklonen unterschiedliche Antikörper produziert werden.

Aus diesem Grund sind Seren immunisierter Tiere immer polyklonal und somit heterogen, sowohl was ihre Spezifität und als auch ihre Zugehörigkeit zu den einzelnen Klassen von Immunglobulinen angeht.

Diese Heterogenität in der Zusammensetzung polyklonaler Antiseren kann daher dazu führen, dass strukturell nahe verwandte Verbindungen, wie beispielsweise Metolachlor und Alachlor bei der Verwendung polyklonaler Antikörper im Rahmen eines Immunassays nicht in ausreichendem Masse differenziert werden können.
Um diese Nachteile der polyklonalen Antiseren zu überwinden wurden in jüngster Zeit vermehrt Anstrengungen unternommen auch für den Agrarsektor monoklonale Antikörper zu entwickeln. So berichten Schlaeppi *et al* (1989) über die Herstellung und Verwendung monoklonaler Antiköprer gegen Atrazin und Hydroxyatrazin in einem Enzym-gekoppelten Immunassay. Die Herstellung monoklonaler Antikörper gegen Metolachlor, die eine ausreichend hohe Affinität gegenüber der Zielsubstanz aufweisen und damit geeignet sind für den erfindungsgemässen Einsatz in einem der bekannten Immunoassays, ist dagegen bisher nicht gelungen.

Die Aufgabe, die es im Rahmen dieser Erfindung zu lösen galt, bestand somit in erster Linie in der Bereitstellung eines einfach handhabbaren, effektiven und in hohem Masse selektiven Immunassays für einen schnellen und zuverlässigen Nachweis von Metolachlor in Boden-, Wasser- und Luftproben sowie in Extrakten aus biologischem Material.

Diese Aufgabe konnte jetzt im Rahmen der vorliegenden Erfindung überraschenderweise gelöst werden und zwar durch die Bereitstellung von monoklonalen Antikörpern mit hoher Spezifität und Affinität für Metolachlor unter Verwendung der an sich bekannten Hybridoma/monoklonalen Antikörper-Technologie.

Die Verwendung hybrider somatischer Zellinien (Hybridomas) als Quelle für Antikörper gegen ganz bestimmte Antigene geht auf die Arbeiten von Köhler und Milstein (Nature, 256: 495-97, 1975) zurück.

Die Antikörper, die sich mit dem dort beschriebenen Verfahren gewinnen lassen, unterscheiden sich sehr stark von denjenigen, die man aus Antiseren konventionell immunisierter Tiere erhält.

Das Prinzip der Hybridoma/monoklonalen Antikörper-Technologie gründet sich auf die Beobachtung, dass beim Verschmelzen zweier somatischer Zellen die resultierende Hybridzelle charakteristische Merkmale beider Eltern typen aufweist.

Im Falle der monoklonalen Antikörperproduktion stammt die Fähigkeit zur Synthese des spezifischen Antikörpers von einer immunkompetenten B-Zelle (gewöhnlich einer Milzzelle), die einem zuvor immunisierten Donor-Tier entnommen wurde, während die Fähigkeit zur kontinuierlichen Zellteilung in Kultur durch den anderen Fusionspartner, eine Tumorzellinie (oft ein Myeloma) beigesteuert wird.

Jede dieser Hybrid-Zellinien synthetisiert ein homogenes Immunglobulin, das nur einen einzigen Vertreter aus der grossen Anzahl möglicher Antikörper repräsentiert, die von einem Tier als Antwort auf ein Antigen *in vivo* synthetisiert werden kann.

Da jeder Immunglobulin-produzierende Klon durch einen einzigen Typ von Antikörpern charakterisiert wird, hat sich die Bezeichnung monoklonale Antikörper eingebürgert.

Die Vorteile monoklonaler gegenüber polyklonalen Antikörpern sind zahlreich:
a) monokolonale Antikörper können in grosser Anzahl und in hohem Reinheitsgrad erhalten werden,
b) die Herstellung monoklonaler Antikörper ist homogen im Hinblick auf die Antigen-Reaktivität und ändert sich auch nicht im Verlaufe der Zeit,
c) monoklonale Antikörper produziernde Hybridomas können über Jahre und Jahrzehnte hinweg aufbewahrt werden, ohne dabei ihre spezifischen Eigenschaften, i.e. die Produktion spezifischer monoklonaler Antikörper, zu verlieren,
d) monoklonale Antikörper sind für die Verwendung als Standardreagentien besser geeignet als polyklonale Antiseren, da letztere negativ beeinflusst werden durch eine grosse Variationsbreite beispielsweise im Hinblick auf
   α) die Blutentnahme immunisierter Tiere zur Gewinnung des Antiserums,
   β) eine ständige Verfügbarkeit von Material für zusätzliche Immunisierungen,
   γ) die begrenzte Lebenszeit der Donortiere.

Monoklonale Antikörper, die mittlerweile gegen eine Vielzahl von Antigenen hergestellt wurden, sind in erster Linie in der medizinischen Diagnostik gut etabliert und aus dieser nicht mehr wegzudenken.

Im Rahmen der vorliegenden Erfindung ist es jetzt erstmals gelungen unter Anwendung der an sich bekannten und zuvor kurz beschriebenen Hybridoma/monoklonalen Antikörper-Technologie monokonale Antikörper mit hoher Spezifität und Affinität für das Herbizid Metolachlor zur Verfügung zu stellen, die aufgrund ihrer hohen Spezifität und der dadurch bedingten geringen Kreuzreaktivität mit strukturell verwandten Verbindungen in hervorragender Weise für eine Verwendung in einem Immunassay zum schnellen und zuverlässigen Nachweis von Metolachlor geeignet sind und die damit auch für eine Differenzierung von Metolachlor gegenüber strukturell verwandten Verbindungen oder inaktiven Metaboliten verwendet werden können.

Die vorliegende Erfindung betrifft somit in erster Linie monoklonale Antikörper und deren Derivate, die eine hohe Spezifität und Affinität gegenüber Metolachlor aufweisen und die im wesentlichen keine Kreuzreaktivität mit den bekanntesten Metolachloranalogen, insbesondere aber keine Kreuzreaktivität mit dem Metolachloranalogen Alachlor zeigen.

Besonders bevorzugt im Rahmen der vorliegenden Erfindung sind monoklonale Antikörper und deren Derivate, die eine hohe Spezifität und Affinität gegenüber Metolachlor aufweisen und die eine Kreuzreaktivität mit den best bekannten, strukturell verwandeten Verbindungen zeigen, die unterhalb von 10%, insbesondere aber unterhalb von 2% und ganze besonders bevorzugt unterhalb von 0.1% liegt.

Unter Derivaten monoklonaler Antikörper sind im Rahmen der vorliegenden Erfindung z.B. Antikörperfragmente zu verstehen, die noch die hohe Spezifität und Affinität für die antigenen Determinanten von Metolachlor besitzen, desweiteren radioaktiv markierte monoklonale Antikörper, die beispielsweise mit radioaktivem Jod (¹²⁵I, ¹³¹I), Kohlenstoff (¹⁴C) Schwefel (³⁵S), Tritium (³H) oder ähnlichem markiert sind, Konjugate monoklonaler Antikörper mit Biotin oder Avidin, mit Enzymen, wie Meerrettichperoxidase, alkalische Phosphatase, β-D-Galaktosidase, Glucoseoxidase, Glucoamylase, Carbonsäureanhydrase, Acetylcholinesterase, Lysozym, Malatdehydrogenase oder Glucose-6-phosphatdehydrogenase, desweiteren Konjugate monoklonaler Antikörper mit biolumineszierenden (z.B. Luciferase), chemolumineszierenden (z.B. Acridiniumester) oder fluoreszierenden (z.B. Phycobiliproteine) Agentien. Ebenso umfasst von der vorliegenden Anmeldung sind bispezifische sowie sog. "cross-linked" Antikörper. Diese beispielhafte Aufzählung möglicher Antikörperderivate dient lediglich der Illustration der vorliegenden Erfindung und soll den Erfindungsgegenstand in keiner Weise limitieren.

Unter der Bezeichnung "im wesentlichen keine Kreuzreaktivität" soll im Rahmen dieser Erfindung verstanden werden, dass die Reaktivität der für Metolachlor spezifischen monoklonalen Antikörper mit unspezifischen Epitopen anderer Verbindungen, insbesondere strukturell verwandter Verbindungen, weniger als 10 %, vorzugsweise aber weniger als 2 % und ganz besonders bevorzugt weniger als 0.1 % beträgt.

Der prozentuale Anteil der Kreuzreaktivität soll im Rahmen dieser Erfindung definitionsgemäss durch die folgende Beziehung wiedergegeben werden:
(Metolachlorkonzentration für 50 % Hemmung/Konzentration der Metolachloranalogen für 50 % Hemmung) x 100.

Eine 50 %ige Hemmung kann beispielsweise mit Hilfe eines kompetitiven ELISA Assays (vgl. Beispiel 8) bestimmt werden. Diese entspricht dann beispielsweise derjenigen Antigenkonzentration, die zu einer 50 %igen Hemmung der Antikörperbindung an das Träger-gebundene Antigen führt.

Ein weiterer Gegenstand der vorliegenden Erfindung betrifft Hybridomazellinien, welche die zuvor näher charakterisierten monoklonalen Antikörper synthetisieren und vorzugsweise in das umgebende Medium sezernieren.

Insbesondere betrifft die vorliegende Erfindung eine Hybridomazellinie, die einen monoklonalen Antikörper produziert, der eine hohe Spezifität und Affinität gegenüber Metolachlor aufweist und der im wesentlichen keine Kreuzreaktivität mit den best bekannten, strukturell verwandten Verbindungen, insbesondere aber keine Kreuzreaktivität mit Alachlor zeigt.

Besonders bevorzugt ist eine Hybridomazellinie, die einen monoklonalen Antikörper synthetisiert und in das umgebende Medium sezerniert, der eine hohe Spezifität und Affinität gegenüber Metolachlor aufweist und der eine Kreuzreaktivität mit den best bekannten, strukturell verwandten Metolachloranalogen von < 10%, insbesondere aber von < 2% und ganze besonders bevorzugt von < 0.1% zeigt.

Ganz besonders bevorzugt ist eine Hybridomazellinie, welche die kennzeichnenden Charakteristika von ECACC 9002 1701 besitzt, sowie deren Klone und Subklone.

Ebenso umfasst von der vorliegenden Erfindung sind Varianten und Mutanten der zuvor näher charakterisierten Hybridomazellinien, die spontan entstehen oder aber artifiziell mit Hilfe bekannter Verfahren hergestellt werden können und die noch die charakteristischen Eigenschaften des Ausgangsmaterials aufweisen, d.h. die noch in der Lage sind die erfindungsgemässen Antikörper oder Derivate davon zu produzieren und ins umgebende Medium zu sezernieren.

Ebenso umfasst von der vorliegenden Erfindung sind Verfahren zur Herstellung besagter Hybridomazellinien sowie Verfahren zur Herstellung besagter monoklonaler Antikörper.

Unter Klonen und Subklonen von Hybridomazellinien sind Hybridomas zu verstehen, die durch wiederholtes Klonieren aus dem Ausgangsklon hervorgehen und die noch die erfindungswesentlichen Merkmale des Ausgangsklons aufweisen.

Ein weiterer Gegenstand dieser Erfindung betrifft ein Verfahren zum immunologischen Nachweis von Metolachlor z.B. in Boden-, Wasser- oder Luftproben sowie in biologischem Material, wie z.B. in pflanzlichen oder tierischen Extrakten, unter Verwendung der erfindungsgemässen monoklonalen Antikörper.

Besonders bevorzugt ist in diesem Zusammenhang ein kompetitiver ELISA, der als direkter oder als indirekter Immunassay Anwendung finden kann.

Ebenso ein Bestandteil der vorliegenden Erfindung sind Mittel für die qualitative und quantitative Bestimmung von Metolachlor in Form gebrauchsfertiger Test-Kits, welche mindestens einen der erfindungsgemässen monoklonalen Antikörper als Reagenz enthalten und die für die Verwendung unter Feldbedingungen für einen schnellen und zuverlässigen Nachweis von Metolachlor geeignet sind.

Die Herstellung der erfindungsgemässen monoklonalen Antikörper erfolgt mit Hilfe an sich bekannter Verfahren, die im wesentlichen auf den von Köhler und Milstein (Nature, 256: 495-497, 1975) entwickelten Methoden beruhen.

Da es sich bei der zu analysierenden Zielsubstanz Metolachlor, für die spezifische monoklonale Antikörper entwickelt werden sollen, um ein relativ kleines und einfaches Molekül handelt, das nach der Applikation in ein Versuchstier alleine nicht in der Lage ist dort eine entsprechende Immunantwort auszulösen, müssen vor der eigentlichen Immunisierung zunächst vorbereitende Massnahmen getroffen werden.

Man bezeichnet solche Verbindungen, die aufgrund ihrer Grösse und einfachen Strukturierung nicht zur Induktion einer Immunreaktion in der Lage sind, als Haptene oder inkomplette Antigene und stellt sie somit den kompletten Antigenen (= Immunogene) gegenüber, die sowohl als Antigen wirken als auch eine Immunantwort zu induzieren vermögen. Solche Haptenmoleküle können an hochmolekulare Verbindungen (Trägermoleküle) konjugiert werden, wodurch sie in ihren Eigenschaften kompletten Antigenen vergleichbar werden, d.h. sie besitzen jetzt die Fähigkeit zur Auslösung einer Immunantwort.

Einige der im Verlaufe der Immunisierungsreaktion gebildeten Antikörper sind dann in der Lage mit spezifischen Epitopen auf dem Haptenmolekül zu reagieren, unabhängig davon, ob das Haptenmolekül alleine oder aber nach wie vor gekoppelt an das Carriermolekül vorliegt.

Unter der im folgenden häufig verwendeten Bezeichnung Hapten soll im Rahmen dieser Erfindung in erster Linie das für die Immunisierung verwendeten Metolachlor-Molekül verstanden werden.

Im Rahmen dieser Erfindung wird daher das als Hapten wirkenden Metolachlor vor der Immunisierung von Versuchstieren an einen hochmolekularen Träger ('Carrier') gekoppelt, der geeignet ist besagtem Metolachlor eine komplette antigene Wirksamkeit zu verleihen.

Unter geeigneten Carrier-Molekülen sind im Rahmen dieser Erfindung in erster Linie makromolekulare Verbindungen zu verstehen, die frei zugängliche reaktive Gruppen für die Kupplungsreaktion mit dem Hapten aufweisen und die in der Lage sind, durch Kupplung an das Hapten, diesem eine immunogene Potenz zu verleihen oder aber dessen bereits vorhandene Immunogenizität zu verstärken.

Besonders bevorzugt im Rahmen dieser Erfindung sind makromolekulare Verbindungen, die frei zugängliche reaktive Aminogruppen enthalten.

Ganz besonders bevorzugt für die erfindungsgemässe Verwendung als Carriermolekül sind lysinreiche Proteine mit einem Molekulargewicht zwischen 10'000 und 1'500'000, wie z.B. "Bovine Serum Albumin" (BSA: MG 66'200), Humanes Serum Albumin (HSA,; MG 58'000) oder "Keyhole Limpet Hemocyanin" (KLH; MG >1'000'000), die kommerziell erhältlich sind und somit in beliebiger Menge zur Verfügung stehen.

Selbstverständlich können im Rahmen der vorliegenden Erfindung auch andere makromolekulare Verbindungen als Carrier-Moleküle verwendet werden, sofern sie die oben genannten Voraussetzungen erfüllen, wie z.B. "Porcine Thyroglobulin", B2 Microglobulin, Hemocyanin, Immunglobuline, Toxine (Cholera-, Tetanus-, Diphtheria-Toxin u.a.), Polysaccharide, Lipopolysaccharide, natürliche oder synthetische Polyadenyl- und Polyuridylsäuren, Polyalanyl- und Polylysin-Polypeptide oder Zellmembrankomponenten, wie beispielsweise Formalin- oder Glutaraldehyd-behandelte Erythrozytenzellmembranen.

Ebenso geeignet für die Verwendung als Carriermolekül in dem erfindungsgemässen Verfahren ist beispielsweise die gereinigte IgG Fraktion gegen Maus IgG (H+L) aus Kaninchen gemäss dem bei H Kawamura und JA Berzofsky (J. Immunol 136: 58, 1986) beschriebenen Verfahren.

Die Konjugation des Haptens an das Trägermolekül kann entweder direkt oder aber vorzugsweise über ein Brückenglied erfolgen, welches gegebenenfalls zunächst an das Haptenmolekül angelagert wird.

Die Kupplung der zu analysierenden Substanz an das Trägermolekül muss dabei in einer Weise erfolgen, dass die relevanten Strukturelemente der Zielsubstanzen frei zugänglich bleiben und somit in der Lage sind eine spezifische Immunantwort auszulösen, d.h. die Bildung spezifischer Antikörper zu induzieren.

Als Brückenglieder für eine Konjugation des Haptens (Metolachlor) an das Carriermolekül kommen in erster Linie Verbindungen in Betracht, die mindestens eine oder aber mehrere reaktive Gruppen enthalten, welche in der Lage sind mit den frei zugänglichen reaktiven Gruppen des Carrier-Moleküls in Wechselwirkung zu treten.

Besonders bevorzugt im Rahmen dieser Erfindung ist die Verwendung von Brückengliedern die zwischen 3 und 10 Brücken-C-atome umfassen und die als reaktive Gruppe(n) eine oder mehrere reaktive Gruppen, wie z.B. Amino-, Carboxyl- oder SH-Gruppe(n) besitzen. Diese reaktiven Gruppen können mit Hilfe an sich bekannter Verfahren mit den reaktiven Gruppen des Hapten- und Carrier-Moleküls zur Reaktion gebracht werden, unter Ausbildung eines Hapten-Carrier-Konjugates.

So ist es beispielsweise möglich ein Brückenglied über eine reaktive Aminogruppe mit Hilfe von Dialdehyden (z.B. Glutardialdehyd) an eine der freien Aminogruppen des Carrier-Moleküls zu binden.

Besitzt das Brückenglied eine reaktive SH-Gruppe, so kann die Konjugation des Haptens an das Carrier-Molekül durch eine Oxidation mit freien SH-Gruppen des Carriers durchgeführt werden.

Besonders bevorzugt im Rahmen dieser Erfindung ist die Verwendung von Brückengliedern mit einer Carboxylgruppe, die mit Hilfe von wasserbindenden Mitteln, wie beispielsweise einem Carbodiimid, vorzugsweise N,N'-Dicyclohexylcarbodiimid, mit einer freien Aminogruppe des Carriermoleküls verknüpft werden kann.

Um das Antigen an das Trägerprotein zu kuppeln, muss somit zunächst ein zu dieser Kupplung befähigtes Metolachlor-Derivat hergestellt werden.

Metolachlorderivate, die im Rahmen der vorliegenden Erfindung Verwendung finden können sind insbesondere solche der Formel (I), 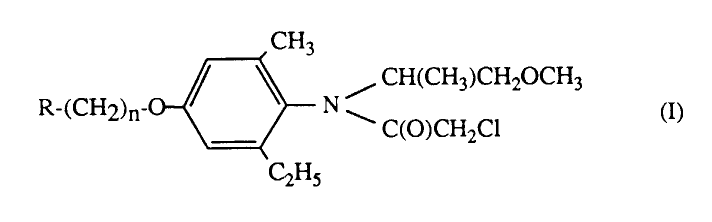 die in 4 Stellung zur Aminofunktion des Metolachlors eine R-(CH₂)ₙ-O- Gruppierung aufweisen, worin
R für COOH, NH₂ oder SH, insbesondere aber für COOH steht und
n eine ganze Zahl von 1 bis 10, vorzugsweise von 1 bis 6 repräsentiert.

Zur Kupplung an ein Trägermolekül befähigte Metolachlorderivate der Formel I können dadurch hergestellt werden, dass man eine Verbindung der Formel II 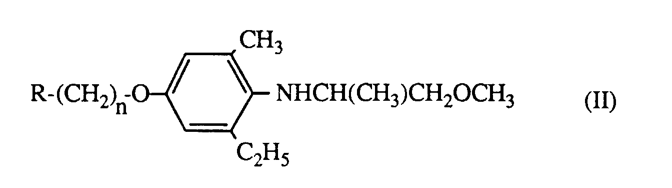 worin R und n die unter Formel I angegebenen Bedeutungen haben, zweckmässigerweise in einem reaktionsinerten Lösungsmittel, vorzugsweise unter milden Bedingungen [-10°C bis + 30°C] mit einem zur N-Acylierung befähigten Chloressigsäurederivat N-acyliert.

Als N-Acylierungsmittel kommen reaktionsfähige Chloressigsäure-Derivate, insbesondere Säurehalogenide [z.B. Chloressigsäurechloride und -bromide], -Ester und -Anhydride in Betracht. Bei dieser Reaktion ist es vorteilhaft die freiwerdende Säure durch ein geeignetes Bindemittel abzufangen. Hierzu eignen sich z.b. organische Basen wie Trialkylamine [Trimethylamin, Triethylamin usw.], Pyridin- und Pyrimidinbasen oder anorganische Basen wie Oxide, Hydroxide [z.B. NaOH oder KOH], Hydrogencarbonate, Carbonate oder Hydride von Alkali- und Erdalkalimetallen. Es ist zweckmässig die Umsetzung in Gegenwart von 2 Äquivalenten des Bindemittels [bezogen auf Produkt I] durchzuführen; als Katalysator lassen sich hierbei 4-Dialkylaminopyridine verwenden.

Die Herstellung von Verbindungen der Formel II erfolgt analog zu der weiter unten für N-Chloracetyl-N-(1-methyl-2-methoxyethyl)-2-methyl-4-(4'-hydroxycarbonylbutoxy)-6-ethylanilin schematisch angegebenen Arbeitsweise.

Ein bevorzugtes Beispiel eines für die Kupplung an ein Trägermolekül geeigneten Metolachlor-Derivates ist das N-Chloracetyl-N-(1-methyl-2-methoxyethyl)-2-methyl-4-(4'-hydroxycarbonylbutoxy)-6-ethylanilin, das wie nachfolgend im einzelnen beschrieben wird, in einem 6-stufigen Verfahren ausgehend vom 3-Ethyl-4-nitroso-5-methylphenol hergestellt werden kann. Das Ausgangsphenol ist bekannt oder lässt sich analog zu bekannten strukturähnlichen Phenolen herstellen.

Das resultierende, zur Kupplung befähigte Metolachlor-Derivat ist neu und stellt durch diese spezifische Kupplungsfähigkeit ein wertvolles Ausgangsmaterial bei der Herstellung monoklonaler Antikörper mit Metolachlorspezifität dar. Es ist daher ein wichtiger Bestandteil vorliegender Erfindung.

Die Durchführung der eigentlichen Kupplungsreaktion erfolgt vorzugsweise mit Hilfe der aktiven Estermethode. Dabei wird das Metolachlorderivat zunächst in einem geeigneten Lösungsmittel solubilisiert. Als geeignete Lösungsmittel kommen insbesondere aprotische Lösungsmittel, die eine geringe Verdampfungsrate aufweisen, wie z.B. N,N-Dimethylformamid (DMF) oder Dimethylsulfoxid (DMSO) in Betracht.

Anschliessend werden die Carboxylgruppen zu einem aktiven Ester derivatisiert, indem das zuvor solubilisierte Metolachlorderivat z.B. mit N-Hydroxysuccinimid, N-Hydroxysulfosuccinimid, N,N'-Dicyclohexylcarbodiimid oder N,N'-Carbonyldiimidazol oder mit Derivaten dieser Verbindungen zur Reaktion gebracht wird.

Der aktive Ester wird dann aus dem Reaktionsgemisch abgetrennt und zu BSA oder KLH zugegeben. Nach einer Inkubationszeit von 0.1 bis 12 Stunden vorzugsweise von 3 bis 5 Stunden wird das Präzipitat entfernt. Der Ueberstand kann dann gegebenenfalls nach Zwischenschaltung weiterer Reinigungsschritte für die eigentliche Immunisierungsreaktion verwendet werden.

Neben der im Rahmen dieser Erfindung bevorzugten aktiven Estermethode, können auch alternative Verfahren für die Kupplung des Haptens an das Carrier-Molekül verwendet werden, wie z.B. die gemischte Anhydridmethode. Dabei wird die Carboxylgruppe des Brückengliedes unter Verwendung von Essigsäureanhydrid oder des Carbodiimidderivates 1-Ethyl-3-(3'-dimethylaminopropyl)carbodiimid an das Carrier-Molekül geknüpft.

Die Immunisierung der Donortiere erfolgt durch eine ein- bis mehrmalige Applikation des an ein hochmolekulares Trägermolekül gekoppelten Haptens. Besonders bevorzugt ist eine 2 bis 3 malige Applikation, die in Abständen von 7 bis 30 Tagen insbesondere aber von 12 bis 16 Tagen erfolgt.

Die im Rahmen der vorliegenden Erfindung bevorzugte Applikationsform ist die Injektion, die sowohl intravenös, intraperitoneal oder subcutan erfolgen kann. Bevorzugt ist eine Kombination von subcutaner und intraperitonealer Injektion.
Das Antigen (Metolachlor-konjugat) liegt in diesem Fall in einem geeigneten Puffer vor, wie z.B. einem PBS-Puffer, der eines der üblicherweise verwendeten Adjuvantien enthält. Besonders bevorzugt im Rahmen dieser Erfindung ist die Verwendung von Freund's Adjuvans.

Nach einer Ruheperiode von 0,5 bis 4 Monaten erfolgt eine weitere einmalige Applikation des Haptenkonjugates in einer Dosierung von 100 µg bis 1000 µg.

In einem Zeitraum von 1 bis 6 Tagen nach der letzten Dosierung werden die Donortiere getötet und es wird eine Milzzellensuspension hergestellt.

Die isolierten Milzzellen werden dabei in einem geeigneten Puffer (z.B. einem BSS-Puffer) suspendiert und bis zu ihrer Fusion mit geeigneten Myeloma-Zellen in Form einer Zellsuspension aufbewahrt.

Anfänglich wurden diese Fusionen dadurch kompliziert, dass auch die Myeloma-Zellinien monoklonale Antikörper synthetisierten, so dass das Hybrid zwei Typen monoklonaler Antikörper produzierte, einen, der seinen Ursprung in der Myeloma-Zelle hatte und einen zweiten, der durch die genetische Information der immunokompetenten Zelle bestimmt wurde.

Im Rahmen der vorliegenden Erfindung werden daher vorzugsweise solche Tumorzellen verwendet, die selbst keine monoklonale Antikörper herstellen können, wie z.B. SP2/0-Ag14 (Shulman *et al,* 1978) oder X63-Ag8.653, wodurch die Analyse der resultierenden Fusionsprodukte sehr stark vereinfacht wird. Für den Fusionserfolg ist es vorteilhaft, wenn die Milzzellen in einem 2 bis 20fachen Ueberschuss im Verhältnis zu den Myelomzellen vorliegen.

Die Fusion von Milz- und Myelomzellen wird in einem speziellen Fusionsmedium durchgeführt, das eine Zusammensetzung aufweist, die für die beabsichtigte Zellfusion optimale Voraussetzungen liefert.

Vorzugsweise handelt es sich bei besagtem Fusionsmedium um eine Pufferlösung, die einen der üblicherweise für die Fusionierung von Zellen verwendeten Fusionspromotoren enthält, wie z.B. Sendai Viren oder andere Paramyxoviren, gegebenenfalls in UV-inaktivierter Form, Calcium-Ionen, oberflächenaktive Lipide, wie z.B. Lysolecithin oder Polyethylenglykol. Besonder bevorzugt im Rahmen dieser Erfindung ist die Verwendung von Polyethylenglykol (PEG), insbesondere von Polyethylenglykol (PEG) mit einem mittleren MG von 600 bis 6000, und in einer Konzentration von 30 % bis 60 %. Besonders bevorzugt ist eine PEG-Konzentration von 40 % - 50 %. Die optimale Fusionstemperatur beträgt zwischen 18°C und 39°C. Besonders bevorzugt ist eine Temperatur von 37°C.

Nach der erfolgten Fusion der immunkompetenten Milzzellen mit den Myeloma-Zellen werden die fusionierten Antikörper-produzierenden Hybridzellen mit Hilfe an sich bekannter Verfahren selektioniert.
Für das Auslesen erfolgreicher Fusionsereignisse (Hybridzellen) aus den 2 Typen von Elternzellen existieren verschiedene Möglichkeiten. Routinemässig werden eine Million und mehr Zellen von jedem Elterntyp in dem Fusionsprotokoll verwendet. Da die Fusion nicht mit einer 100 %igen Frequenz erfolgt, kann es zu einem schwierigen Unterfangen werden, die Fusionsprodukte von der grossen Ueberzahl nicht fusionierter oder mit sich selbst fusionierter Eltern zellen abzutrennen.

Wie bereits zuvor erwähnt, entstehen die Hybridomazellen durch Fusion kurzlebiger Antikörper produzierender (Milz) B-Zellen sowie langlebiger Myeloma Zellen.

Das gewünschte Resultat ist eine langlebige Zellinie, die Antikörper produziert. Da die Milzzellen nur eine begrenzte Lebenszeit in Kultur besitzen, kann man daher im Prinzip einfach abwarten, bis alle nicht-fusionierten sowie alle mit sich selbst fusionierten Milzzellen abgestorben sind. Dann bleibt jedoch immer noch die Aufgabe bestehen, die langlebigen Antikörper-produzierenden Zellen von den langlebigen nicht Antikörper-produzierenden Zellen abzutrennen.

Ein gängiges Selektionssystem basiert auf der Verfügbarkeit oder Nichtverfügbarkeit des Enzyms Hypoxanthinguaninphosphoribosyltransferase (HGPRT). Dieses Enzym ist Bestandteil des Purin "Salvage Pathway" in Säugerzellen. Diese Zellen sind darüberhinaus auch in der Lage Purine *de novo zu* synthetisieren.

Unter normalen Umständen arbeiten wahrscheinlich beide Synthese-Wege bis zu einem gewissen Grade parallel.

Falls eine Zelle jedoch kein HGPRT besitzt, ist der "Salvage Pathway" blockiert und die Purine müssen aus nicht-Purin Material hergestellt werden.

Für die Selektion HGPRT-negativer Myelomazellen verwendet man in der Regel sog. Purin-Antimetaboliten, wie z.B. das 8-Azaguanin, das dem Purin Guanin strukturell sehr ähnlich ist und dieses daher in einigen seiner normalen Reaktionen ersetzen kann.

Azaguanin wird in die DNA eingebaut, was zu einer Beeinträchtigung des normalen Wachstumsverhaltens und schliesslich zum Zelltod führt. Da Azaguanin über den "Salvage Pathway" ersetzt werden muss, können alle diejenigen Zellen, die keine HGPRT Aktivität besitzen, Azaguanin nicht verwerten und daher in dessen Gegenwart wachsen.

Ein selektives System, das mit demselben Enzym aber unter umgekehrten Vorzeichen arbeitet, indem in diesem Fall HGPRT positive Zellen selektiert werden, ist bei J.W. Littlefield (1964) beschrieben.

Dieses Selektionssystem basiert auf der Verwendung des sogenannten HAT-Mediums, das u.a. Hypoxanthin, Aminopterin und Thymidin (HAT-Medium) als Bestandteile enthält. Aminopterin ist ein Antimetabolit, der die *de novo* Purin-Synthese sowie die Methylierung von Desoxyuridylat zu Thymidylat hemmt.

Hypoxanthin kann als Hilfspurin fungieren für den Fall, dass Aminopterin die *de novo* Purin-Synthese blockiert, während Thymidin die Notwendigkeit einer Methylierung von Desoxyuridylat überflüssig macht.

Daher werden in Gegenwart von Aminopterin alle HGPRT positiven Zellen proliferieren, während die HGPRT negativen Zellen absterben.

In dem Hybridsystem, das im Rahmen dieser Erfindung für die Selektion verwendet wird, sind die Myeloma-Zellen vorzugsweise resistent gegenüber Azaguanin und empfindlich gegenüber Aminopterin, d.h. sie sind HGPRT negativ. Die Antikörper-produzierenden Zellen dagegen sind HGPRT positiv.

Durch Fusion der Zellen und Kultivierung in einem HAT-Medium, können die erfolgreich miteinander fusionierten Zellen selektiert werden, da die Myeloma-Zellen, die für die Proliferation verantwortlich sind, nur in Gegenwart einer HGPRT-Aktivität wachsen können und diese Aktivität von der HGPRT-positiven Zellinie geliefert werden muss.

Die HGPRT-positiven Antikörper-produzierenden Zellinien werden in diesem Medium zwar nicht abgetötet. Sie überleben aber nur eine bestimmte Zeit, und sind nicht in der Lage zu proliferieren.

Damit wird durch Fusion der Zellen in einem HAT-Medium ein System geschaffen, in welchem zwar die Myeloma Zellen und die Antikörper produzierenden Zellen für einen Zeitraum wachsen können, der ausreicht für die Produktion von Hybrid-Zellen, in dem aber nur die Hybrid-Zellen überleben und proliferieren können.

In einer besonderen Ausführungsform der vorliegenden Erfindung werden die fusionierten Hybridzellen in Gegenwart von zuvor aus dem Peritoneum unbehandelter, nicht-immunisierter Versuchstiere isolierten Macrophagen, sogenannter "Feederzellen", kultiviert. Für die Kultivierung und die Selektion der fusionierten Hybridzellen wird die Zellsuspension in mehrere Aliquots aufgeteilt und die einzelnen Aliquots werden ständig auf die Ausbildung von Hybridzellkulturen sowie auf die Bildung von Antikörpern hin untersucht.

Besonders bevorzugt im Rahmen dieser Erfindung ist die Kultivierung der fusionierten Hybridzellen auf Mikrotiterplatten.

Dabei wird die nach der Fusion erhaltene Zellsuspension auf die einzelnen Vertiefungen einer Mikrotiterplatte verteilt und für einen Zeitraum von 7 bis 30 Tagen unter geeigneten, das Wachstum der fusionierten Hybridzellen fördernden Bedingungen (z.B. HAT/HT-Medien) kultiviert.

Die Ueberstände gewachsener Hybridkulturen werden ständig auf die Bildung von Antikörpern hin untersucht.

Positive Hybridzellkulturen werden dann mit Hilfe bekannter Verfahren, vorzugsweise aber mit Hilfe des limitierenden Verdünnungsverfahrens vereinzelt und anschliessend in geeigneten Kultivierungsmedien kloniert.

Die Ueberstände der gewachsenen Zellklone werden ebenfalls auf die Bildung von Antikörpern hin überprüft.

Das Screening der gemäss der vorangegangenen Beschreibung hergestellten erfindungsgemässen Hybridoma-Zellklone auf die Bildung geeigneter monoklonaler Antikörper erfolgt vorzugsweise mit Hilfe eines der üblicherweise für diesen Zweck verwendeten Immunassays, wie z.B. einem Enzym-gekoppelten Immunassay oder einem Radioimmunassay.

Beim Enzym-gekoppelten Immunassay werden die zuvor näher charakterisierten Hapten-Konjugate zunächst an einen festen Träger adsorbiert. Die verbleibenden freien Bindungsstellen werden anschliessend durch Zugabe von Carriermolekülen abgesättigt und damit blockiert.

Zum Nachweis monoklonaler Antikörper werden Aliquots der Ueberstände besagter Hybridoma-Zellklone mit den Träger-gebundenen Haptenkonjugaten inkubiert.

Ein weiterer Gegenstand der vorliegenden Erfindung betrifft die Herstellung monoklonaler Antikörper unter Verwendung an sich bekannter Verfahren, die dadurch gekennzeichnet sind, dass man die zuvor näher charakterisierten erfindungsgemässen Hybridomazellinien oder aber Klone oder Subklone davon, welche die erfindungsgemässen Antikörper synthetisieren und in das umgebende Medium sezernieren, *in vitro* oder *in vivo* mit Hilfe bekannter Verfahren kultiviert.

Die *in-vitro*-Kultivierung der erfindungsgemässen Hybridoma-Zellen erfolgt in geeigneten Kultivierungsmedien, insbesondere in den üblicherweise verwendeten, standardisierten Kulturmedien wie z.B. Dulbecco's Modified Eagle Medium (DMEM) oder RPMI 1640 Medium, die gegebenenfalls durch Zugabe von Säuger-Seren, wie z.B. Foetales Kälberserum, oder durch wachstumsfördernde Zusätze und Spurenelemente ergänzt werden können.

Die Isolierung der monoklonalen Antikörper beginnt vorzugsweise mit einer Präzipitation der Immunglobulinfraktion aus den jeweiligen Ueberständen der Hybridomakulturen, z.B. mit Hilfe von Ammoniumsulfat. Es schliessen sich weitere Aufarbeitungs- und Reinigungsschritte an, die dem Fachmann auf diesem Gebiet bekannt sind und die beispielsweise die Verwendung chromatographischer Methoden, wie z.B. Gelfiltration, Ionenaustausch-Chromatographie, DEAE-Cellulose Chromatographie, Protein A oder Immunaffinitätschromatographie miteinschliessen.

Grosse Mengen der erfindungsgemässen monoklonalen Antikörper können aber auch mit Hilfe von *in vivo* Verfahren gewonnen werden.

So ist es beispielsweise möglich Antikörper-produzierende Hybridoma-Zellklone in geeignete Säugetiere zu injizieren, welche die Entwicklung von Antikörper-produzierenden Tumoren in den behandelten Tieren induzieren. Nach einen Zeitraum von 1 bis 3 Wochen können die Antikörper aus den Körperflüssigkeiten der so behandelten Tiere isoliert werden.

In einer besonderen Ausführungsform der vorliegenden Erfindung wird weiblichen Balb/c Mäusen, die gegebenenfalls mit einem Kohlenwasserstoff wie z.B. Pristan vorbehandelt wurden, ein erfindungsgemässer Hybridoma-Zellklon intraperitoneal injiziert.
Ein bis drei Wochen nach der Injektion des Hybridoma-Zellklons wird die Ascitesflüssigkeit gesammelt und bis zur weiteren Aufbereitung aufbewahrt.

Die Isolierung der monoklonalen Antikörper erfolgt in genau analoger Weise zu der zuvor beschriebenen Isolierung aus den Ueberständen *in vitro* kultivierter Hybridomas.

Ein weiterer Gegenstand der vorliegenden Erfindung betrifft die Verwendung der erfindungsgemässen Antikörper in einem der gebräuchlichen Immunassays zum Nachweis von Metolachlor sowie zur Differenzierung von Metolachlor gegenüber strukturell verwandten Verbindungen, insbesondere aber gegenüber dem Metolachloranalogen Alachlor in Boden-, Luft- und Wasserproben sowie gegebenenfalls in Extrakten aus Pflanzen oder anderem biologischem Material.

Die erfindungsgemässen monoklonalen Antikörper können somit in allen bekannten Immunassays verwendet werden, die auf der spezifischen Bindung zwischen Antigen und dem entsprechenden monoklonalen Antikörper aufbauen, wie z.B. in einem Radioimmunassay (RIA), einem Enzym-gekoppelten Immunassay (ELISA), einem Immunfluoreszenz-Test etc.

Beim RIA Test kann der erfindungsgemässe monoklonale Antikörper als solcher oder aber in Form eines radioaktiv markierten Derivates verwendet werden. Dabei können alle bis heute bekannten Modifikationen des RIA Tests für den Nachweis der im Rahmen dieser Erfindung relevanten Zielsubstanzen verwendet werden, wie z.B. ein RIA Test in homogener oder fester Phase, ein heterogener RIA Test sowie ein einfacher oder doppelter ('Sandwich') RIA Test mit direktem oder indirektem (kompetitiven) Nachweis des Antigens. Das gleiche gilt auch für die Verwendung eines enzymgekoppelten Immunassays.

Bevorzugt im Rahmen dieser Erfindung ist die Verwendung eines erfindungsgemässen monoklonalen Antikörpers in einem kompetitiven Immunassay zum Nachweis von Metolachlor.

Das Prinzip des kompetitiven Immunassays beruht auf einer Konkurrenz zwischen einem markierten bzw. einem an einen festen Träger gebundenen Antigen und einem freien Antigen um die relevanten Bindungsstellen auf dem Antikörpermolekül.

Grundsätzlich unterscheidet man zwei Möglichkeiten zur Durchführung dieses kompetitiven Immunassays.
a) Das erste Verfahren beruht auf der Konkurrenz zwischen dem an einen festen Träger gebundenen Antigen und freiem Antigen um die freien Bindungsstellen auf dem Antikörper, der mit einem Marker versehen ist. Die Bindung des Antigens an einen festen Träger kann dabei entweder direkt oder aber über ein Carriermolekül erfolgen.
   Die Bestimmung der Konzentration an freiem Antigen erfolgt in diesem Fall über die Abnahme des markierten, an das Träger-fixierte Antigen gebundenen Antikörpers.
   Diese Abnahme ist proportional der in der Probe enthaltenen Menge an freiem Antigen.
b) Ein alternatives Verfahren basiert darauf, dass freies und markiertes Antigen miteinander um die relevanten Bindungsstellen des Antikörpers konkurrieren, der in diesem Fall an einen festen Träger gebunden vorliegt.
   Die Bestimmung der Konzentration an freiem Antigen erfolgt über die Abnahme an markiertem Antigen, die in Abhängigkeit von der Konzentration an freiem Antigen variiert.

Als festes Trägermaterial, das für die Bindung des Antigens oder des Antikörpers geeignet ist, kommen beispielsweise die Plastikoberfläche einer Mikrotiterplatte oder eines Teströhrchens, die Oberfläche von Kugeln aus Polystyrol, Polypropylen, Polyvinylchlorid, Glas oder Kunststoff oder aber die Oberfläche von Filterpapier-, Dextran-, Cellulose- oder Nitrozellulose-Streifen oder ähnliche Materialien in Betracht. Diese werden mit einem der erfindungsgemässen monoklonalen Antikörper oder einem Antigen überzogen, wobei die Bindung an das Trägermaterial durch einfache Adsorption oder aber gegebenenfalls nach vorangegangener Aktivierung des Trägermaterials mit z.B. Glutaraldehyd oder Cyanogenbromid vermittelt werden kann.

Besonders bevorzugt im Rahmen dieser Erfindung ist die Verwendung des erfindungsgemässen monoklonalen Antikörpers in einem Enzym-gekoppelten Immunassay [ELISA ('Enzyme Linked Immuno Sorbent Assay')]. Dabei kann der erfindungsgemässe monoklonale Antikörper als solcher oder in Form eines Enzym-gekoppelten Derivates verwendet werden.

Der ELISA Assay basiert entweder auf der Verwendung eines Enzym-gekoppelten Derivates des erfindungsgemässen Antikörpers oder aber von an sich bekannten Enzym-gekoppelten Antikörpern, die ein Epitop eines erfindungsgemässen Antikörpers erkennen und daran binden.

Besonders bevorzugt im Rahmen dieser Erfindung ist die Verwendung eines indirekten ELISA-Assays, bei dem eines der zuvor beschriebenen Trägermaterialien zunächst mit einem Antigen, insbesondere einem Konjugat aus Hapten und einem hochmolekularen Trägermolekül wie z.B. BSA oder KLH, beschichtet wird. Bevorzugt im Rahmen der vorliegenden Erfindung ist eine Konjugatkonzentration von 10 ng/100 µl Puffer bis 200 ng/100 µl Puffer, vorzugsweise von 30 ng/100 µl Puffer bis 100 ng/100 µl Puffer und ganz besonders bevorzugt von 40 ng/100 µl Puffer bis 60 ng/100 µl Puffer.

Das trägergebundene Antigen wird anschliessend mit einer Testlösung inkubiert, die das nachzuweisende Antigen sowie einen der erfindungsgemässen Antikörper enthält. Das nachzuweisende Antigen kann dabei entweder in freier Form oder aber als Bestandteil einer Wasser- oder Bodenprobe vorliegen.

Nach einer Inkubationszeit von 10 Minuten bis 2 Stunden wird der gesamte Ansatz mit einem enzymmarkierten Antikörper inkubiert, der den erfindungsgemässen monoklonalen Antikörper erkennt und an diesen bindet. Ein Beispiel eines solchen enzymmarkierten Antikörpers ist ein Phosphatase-markiertes Ziegen anti-Schaf Immunglobulin, oder ein entsprechender Ziegen anti-Maus Antikörper, die kommerziell bezogen werden können.

Die Menge des gebundenen Antikörperproteins lässt sich anhand einer Enzym-Substrat-Reaktion, z.B. mit Hilfe spektroskopischer Verfahren bestimmen.

Ebenfalls bevorzugt im Rahmen dieser Erfindung ist ein direkter ELISA-Test, der auf der Konkurrenz von markiertem sowie von freiem Antigen um den an eines der zuvor genannten Trägermaterialien gebundenen Antikörper beruht.

Die Markierung des Antigens kann mit Hilfe bekannter Verfahren durchgeführt werden unter Verwendung der in der immunologischen Diagnostik üblicherweise verwendeten Marker.
Bevorzugt im Rahmen der vorliegenden Erfindung ist die Verwendung eines Enzym-Markers wie z.B. der alkalischen Phosphatase, der mit dem nachzuweisenden Antigen verknüpft wird.

Der Anteil an freiem Antigen, der in einer bestimmten Probe vorliegt, kann dann sehr einfach anhand der Abnahme an markiertem Antigen bestimmt werden, die umso grösser ist, je mehr freies Antigen in der Probe enthalten ist.

Die vorliegende Erfindung betrifft weiterhin Mittel für die qualitative und quantitative Bestimmung von Metolachlor in Form eines Test-Kits, die neben den erfindungsgemässen monoklonalen Antikörpern und/oder deren Derivaten, gegebenfalls auch noch andere monoklonale oder polyklonale Antikörper, insbesondere aber markierte monoklonale oder polyklonale Antikörper, sowie weitere Zusätze enthalten können.

Besonders bevorzugt im Rahmen dieser Erfindung sind Test-Kits, die auf einem der üblicherweise verwendeten Immunassays basieren, ausgewählt aus der Gruppe bestehend aus Radioimmunassay, Enzym-gekoppelter Immunassay und Chemilumineszenzassay. Ganz besonders bevorzugt sind Test-Kits, bei denen der Nachweis von Metolachlor auf einem kompetitiven Immunassay, insbesondere aber auf einem Enzym-gekoppelten direkten oder indirekten Immunassay (ELISA) beruht.

Test-Kits für einen radioimmunologischen Nachweis von Metolachlor, können beispielsweise die folgenden Bestandteile enthalten:
(a) ein geeignetes Trägermaterial, das unbeschichtet oder aber mit einem der erfindungsgemässen Antikörper oder einem Antigenkonjugat beschichtet sein kann;
(b) gegebenenfalls gefriergetrocknete oder konzentrierte Lösungen eines der erfindungsgemässen Antikörper und/oder eines radioaktiv markierten Derivates davon oder radioaktiv markiertes Antigen oder standardisierte Lösungen des Antigens;
(c) Pufferlösungen und
(d) gegebenenfalls Polypeptide, Detergentien und weitere Zusätze, die beispielsweise eine unspezifische Adsorption und Aggregatbildung verhindern sowie
(e) Pipetten, Reaktionsgefässe, Eichkurven, Beipackzettel etc.

Test-Kits für den immunologischen Nachweis von Metolachlor, die auf einem Enzymgekoppelten Immunassy (ELISA) basieren, können beispielsweise die folgenden Bestandteile enthalten:
(a) ein geeignetes Trägermaterial, das unbeschichtet oder aber mit einem der erfindungsgemässen Antikörper oder einem Antigenkonjugat beschichtet sein kann;
(b) gegebenenfalls gefriergetrocknete oder konzentrierte Lösungen eines der erfindungsgemässen Antikörper und/oder eines zweiten Enzymmarkierten monoklonalen oder polyklonalen Antikörpers, der gegen das zu bestimmende Antigen oder einen das Antigen erkennenden Antikörper gerichtet ist;
(c) Enzymsubstrate in fester oder gelöster Form;
(e) das Antigen oder standardisierte Lösungen des Antigens;
(f) Pufferlösungen;
(g) gegebenenfalls Polypeptide, Detergentien und weitere Zusätze, die beispielsweise eine unspezifische Adsorption und Aggregatbildung verhindern sowie
(h) Pipetten, Reaktionsgefässe, Eichkurven, Farbtafeln, Beipackzettel, etc.

Ein Test-Kit für den Nachweis von Metolachlor, der auf einem Chemilumineszenztest basiert, kann beispielsweise die folgenden Bestandteile enthalten:
(a) ein geeignetes Trägermaterial, das unbeschichtet oder aber mit einem der erfindungsgemässen Antikörper oder einem Antigenkonjugat beschichtet sein kann;
(b) gegebenenfalls gefriergetrocknete oder konzentrierte Lösungen eines der erfindungsgemässen Antikörper und eines zweiten polyklonalen Antikörpers, der in der Lage ist, den erfindungsgemässen ersten Antikörper zu erkennen und der mit einem chemilumineszierenden Marker verknüpft ist;
(c) Lösungen enthaltend eine Komponente, die die Emission von Licht auslöst, wie z.B. H₂O₂ und NaOH;
(d) Pufferlösungen;
(e) gegebenenfalls Polypeptide, Detergentien und weitere Zusätze, die eine unspezifische Adsorption und Aggregatbildung verhindern sowie
(f) Pipetten, Reaktionsgefässe, Beipackzettel, etc.

Trägermaterialien, die im Rahmen der vorliegenden Erfindung Verwendung finden können, umfassen in erster Linie unlösliche, polymere Materialien, ausgewählt aus der Gruppe bestehend aus Polystyrol, Polyethylen, Polypropylen, Polyester, Polyacrylnitril, Polyvinylchlorid, Polyacrylamid, Nitrocellulose, quervernetztes Dextran, fluorierte Harze, Agarose, quervernetzte Agarose, Polysaccharide, etc. Daneben sind aber auch andere Materialien denkbar, wie z.B. Glas, Metall, Netzgewebe auf Nylonbasis, etc.

Die zuvor im einzelnen genannten Trägermaterialien können sehr unterschiedlich ausgestaltet sein und in Abhängigkeit vom jeweils angestrebten spezifischen Verwendungszweck sehr verschiedenartige Formen aufweisen. Diese umfassen beispielsweise Schalen, Kugeln, Platten, Stäbchen, Zellen, Fläschchen, Röhrchen, Fasern, Netze, etc.

Häufige Verwendung bei der Herstellung von Test-Kits finden beispielsweise Mikrotiterplatten aus durchsichtigen Plastikmaterialien, wie z.B. Polyvinylchorid oder Polystyrol, die unbeschichtet oder aber mit einem der erfindungsgemässen Antikörper, mit freiem Antigen oder einem Antigenkonjugat beschichtet sein können. Ebenso verwendet werden Kügelchen, Röhrchen oder Stäbchen aus Polystyrol sowie Polystyrollatex, wobei das umgebende Latexmaterial via Zentrifugation von den Polystyrolpartikeln abgetrennt werden kann.

Einen weiteren Bestandteil des erfindungsgemässen Testkits bilden Marker oder Indikatoren, mit deren Hilfe es möglich ist, das Vorliegen einer Komplexbildungsreaktion, insbesondere aber einer Immunreaktion nachzuweisen, die vorzugsweise zu einem Antigen-Antikörper-Komplex oder aber zu einem Ligand-Rezeptor-Komplex führt, wobei neben qualitativen gegebenenfalls auch quantitative Aussagen über das nachzuweisende Antigen möglich sind. Als Marker oder Indikatoren kommen sowohl einzelne Atome als auch Moleküle in Betracht, die entweder direkt oder aber indirekt an der Erzeugung eines nachweisbaren Signals beteiligt sein können. Diese Marker oder Indikatoren können entweder direkt mit dem nachzuweisenden Antigen oder mit einem der erfindungsgemässen monoklonalen Antikörper verknüpft sein oder aber in diese eingebaut vorliegen. Sie können aber auch als Einzelsubstanzen oder als Bestandteil einer separaten Verbindung vorliegen, die weder das nachzuweisende Antigen selbst noch einer der erfindungsgemässen monoklonalen Antikörper ist, die ihrerseits aber in der Lage ist mit dem Rezeptormolekül zu reagieren, z.B. in Form einer Komplexbildung.

Bei diesen separat vorliegenden Verbindungen handelt es sich vorzugsweise um ein zweites Antikörpermolekül, das sowohl monoklonalen als auch polyklonalen Ursprungs sein kann, um ein Komplementprotein oder Fragmente davon, um *S.aureus* protein A, etc. Diese separaten Verbindungen erkennen und binden spezifisch an ein Rezeptormolekül, wie z.B. das nachzuweisende Antigen oder einen der erfindungsgemässen monoklonalen Antikörper, vorzugsweise aber an ein Rezeptormolekül, das in Form eines Komplexes vorliegt.

In vielen Fällen sind weitere, zusätzliche Reagentien notwendig, die dann erst im Zusammenwirken mit dem Marker zu einem nachweisbaren Signal führen. Dies gilt insbesondere dann, wenn Enzyme involviert sind.

Marker oder Indikatoren, die im Rahmen der vorliegenden Erfindung verwendet werden können, sind dem Fachmann auf dem Gebiet der Immunologie und Immunchemie bestens bekannt. Sie umfassen beispielsweise radioaktiv markierte Elemente oder Substanzen, Enzyme oder chemilumineszierende Substanzen. Die folgende Aufzählung möglicher Marker oder Indikatoren soll lediglich dazu dienen, die grosse Vielfalt der verwendbaren Substanzen und Reagentien beispielhaft zu veranschaulichen, ohne dadurch jedoch den Erfindungsgegenstand in irgendeiner Weise einzuschränken.

Geeignete Marker oder Indikatoren sind beispielsweise innerhalb der Gruppe der radioaktiven Elemente zu finden. Dabei sind insbesondere solche Elemente bevorzugt, die entweder selbst y Strahlen emittieren, wie z.B. ¹²⁴J, ¹²⁵J, ¹²⁸J, ¹³²J, ⁵¹Cr oder aber eine Emittierung dieser Strahlen induzieren, wie z.B. ¹¹C, ¹⁸F, ¹³N. Ebenfalls geeignet sind sog. β-Stahler wie ¹¹¹In, ¹⁴C und ³H.

Weitere geeignete Marker umfassen chemilumineszierende Substanzen, insbesondere aber fluoreszierende Substanzen, die sehr einfach auf chemischem Wege mit dem Antigen oder einem Antikörper verbunden werden können ohne diesen zu denaturieren. Das entstehende Fluorochrom lässt sich sehr einfach mit Hilfe fluorometrischer Verfahren nachweisen. Im einzelnen zu nennen sind an dieser Stelle Fluorochrome ausgewählt aus der Gruppe bestehend aus Fluoresceinisocyanat, Fluoresceinisothiocyanat, 5-Dimethylamino-1-naphthalinsulfonylchlorid, Tetramethylrhodaminisothiocyanat, Lissamin, Rhodamin 8200 Sulfonylchlorid, etc.

Weitere fluoreszierende Agentien sowie eine Beschreibung von Analysetechniken findet sich bei DeLuca, "Immunofluorescence Analysis", in: Antibody As a Tool, Marchalonis *et al,* John Wiley & Sons, Ltd., pp 189-231 (1982).

Besonders bevorzugt im Rahmen dieser Erfindung ist die Verwendung von Enzymen als Marker- oder Indikatorsubstanzen, wie z.B. Meerrettichperoxidase, alkalische Phosphatase, β-D-Galaktosidase, Glucoseoxidase, Glucoamylase, Carbonsäureanhydrase, Acetylcholinesterase, Lysozym, Malatdehydrogenase, Glucose-6-phosphatdehydrogenase, etc. Bei der Verwendung von Enzymen als Markersubstanzen ist es notwendig zusätzliche Reagentien zuzugeben, die es erlauben, die Bildung eines Immunkomplexes über die Enzymaktivität zu verfolgen sowie gegebenenfalls ein Stop-Reagenz, mit welchem die Enzymreaktion beendet werden kann.

Besonders bevorzugt sind dabei Reagentien, die zu einer Farbreaktion führen. Im Falle der Meerrettichperoxidase sei an dieser Stelle beispielhaft Hydrogenperoxid genannt, das in Kombination mit einem zusätzlichen, oxidierten Farbstoffprecursor wie z.B. Diaminobenzidin oder o-Phenylendiamin, zu einer Braun bzw. Gelbfärbung führt. Bei Verwendung der Glucoseoxidase als Markersubstanz kann beispielsweise 2,2'-Azino-di-(3-ethyl-benzthiazolin-6-sulfonsäure) [ABTS] als Substrat eingesetzt werden.

Ein weiterer Gegenstand der vorliegenden Erfindung betrifft somit die Verwendung von Test-Kits, die zumindest einen der erfindungsgemässen monoklonalen Antikörper als Reagenz enthalten, zum schnellen und effektiven, qualitativen und/oder quantitativen Nachweis von Metolachlor sowie zur Differenzierung von Metolachlor gegenüber den best bekannten, strukturell verwandten Verbindungen, insbesonder aber gegenüber dem Metolachloranalogen Alachlor.

### 1. NICHTLIMITIERENDE AUSFÜHRUNGSBEISPIELE

### Beispiel 1: Synthese eines Metolachlor-Derivates

### 1.1: Herstellung des zur Kupplung an das Trägerproteinbefähigten N-Chloracetyl-N-(1-methyl-2-methoxyethyl)-2-methyl-4-(4'-hydroxycarbonylbutoxy)-6-ethylanilin

### Stufe 1: Herstellung von 3-Ethyl-4-nitroso-5-methylphenol

102 g (749 mMol) 3-Ethyl-5-methylphenol werden in 600 ml Ethanol vorgelegt. Unter kräftigem Rühren lässt man 600 ml konzentrierte Salzsäure zutropfen, wobei das Reaktionsgefäss unter Kühlung auf Raumtemperatur gehalten wird. Anschliessend wird das Reaktionsgemisch auf 0°C abgekühlt. Bei dieser Temperatur lässt man 77,5 g (1123,5 mMol) Natriumnitrit in 78 ml deionisiertem Wasser zutropfen. Das resultierende Gemisch wird noch 2 Stunden bei 5°C weitergerührt und anschliessend auf 3 Liter Eiswasser gegossen, wobei die Titelverbindung auskristallisiert. Sie wird nach dem Abfiltrieren mehrfach mit Eiswasser gewaschen und aus Methanol umkristallisiert. Ausbeute 88,7 g (71,8 % d. Th.); Fp. 138°C (unter Zersetzung).

### Stufe 2: Herstellung von 3-Ethyl-4-amino-5-methylphenol

88,7 g (537,6 mMol) des nach Stufe 1 hergestellten 3-Ethyl-4-nitroso-5-methylphenols werden bei 20-25°C in 1 Liter Tetrahydrofuran (THF) mit molekularem Wasserstoff bei 5 bar katalytisch hydriert. Als Katalysator dienen 10 g 5%-iges Palladium auf Aktivkohle (Pd-C). Die Wasserstoffaufnahme beträgt 77 % d. Th. Der Katalysator wird abfiltriert, das Filtrat eingedampft und der Rückstand aus Methanol umkristallisiert. Ausbeute 51,2 g (63,2 % d. Th.); Fp. 167-170°C.

### Stufe 3: Herstellung von 2-Methyl-4(4'-ethoxycarbonylbutoxy)-6-ethylanilin

Es werden 500 ml Dimethylsulfoxid (DMSO) bei Raumtemperatur vorgelegt und unter kräftigem Rühren 52,1 g (338,6 mMol) des nach Stufe 2 hergestellten 3-Ethyl-4-amino-5-methylphenols in kleinen Portionen zugegeben. Das Gemisch wird auf 15°C abgekühlt. Dann lässt man 33,5 g (507,9 mMol) wässrige 85%-ige Kaliumhydroxidlösung zutropfen, so dass die Reaktionstemperatur 20°C nicht übersteigt. Anschliessend lässt man über einen Zeitraum von 70 Minuten 82,7 ml (507,9 mMol) 5-Bromvaleriansäureethylester zutropfen, wobei man die Reaktionstemperatur bei 25°C hält. Nachdem sich eine homogene Lösung gebildet hat, wird diese auf ein Gemisch von 130 ml 4M Salzsäure und Eis gegeben und gut gerührt. Das wässrige Gemenge wird mit Diethylether gewaschen.

Die wässrige Phase wird dann mit 2M NaOH alkalisch eingestellt, mit Diethylether extrahiert, die Etherphase zweimal mit Wasser und dann mit gesättigter Kochsalzlösung gewaschen, über Natriumsulfat getrocknet und eingeengt. Man erhält 67,5 g eines viskosen Oels, das mittels Vakuumdestillation gereinigt wird. Ausbeute 31,7 g (33,7 % d. Th.), Kp. 151-152°C/10⁻² Torr.

### Stufe 4: Verfahren zur Herstellung von N-(1-Methyl-2-methoxyethyl)-2-methyl-4-(4'-ethoxycarbonylbutoxy)-6-ethylanilin

30,4 g (108,8 mMol) des nach Stufe 3 hergestellten 2-Methyl-4(4'-ethoxycarbonylbutoxy)-6-ethylanilins werden in 300 ml Methanol gelöst und mit 0,3 g konzentrierter Schwefelsäure versetzt. Zu der Lösung lässt man unter Rühren 20 g (163,2 mMol) 72%-iges Methoxyaceton in 100 ml Methanol unter Raumtemperatur (RT) zutropfen. Dann erfolgt katalytische Hydrierung mit molekularem Wasserstoff. Als Katalysator dient 1,5 g 5%-iges Palladium auf Aktivkohle. Nach 8 Stunden bei 5 bar und Temperaturen von 40-45°C beträgt die Wasserstoffaufnahme 106 % d. Th. Der Katalysator wird abfiltriert; der Rückstand in Diethylether aufgenommen; die Etherphase mit nacheinander Natriumbicarbonatlösung, Eiswasser und gesättigter Kochsalzlösung gewaschen, über Natriumsulfat getrocknet und eingedampft. Ausbeute 29 g (75,9 % d. Th.) in Form eines viskosen, hellbraunen Oels.

### Stufe 5: Herstellung von N-(1-Methyl-2-methoxyethyl)-2-methyl-4-(4'-hydroxycarbonylbutoxy)-6-ethylanilin

19 g (54 mMol) des nach Stufe 4 hergestellten N-(1-Methyl-2-methoxyethyl)-2-methyl-4-(4'-ethoxycarbonylbutoxy)-6-ethylanilins werden bei Raumtemperatur mit 150 ml 2M Kaliumhydroxydlösung versetzt. Man lässt die resultierende Emulsion ca. 20 Stunden unter Rühren bei Raumtemperatur zu Ende reagieren. Das Reaktionsgemisch wird anschliessend mit Salzsäure auf pH 7 eingestellt und zweimal mit Diethylether extrahiert. Die Etherphasen werden vereinigt, mit gesättigter Kochsalzlösung mehrfach gewaschen, über Natriumsulfat getrocknet und eingedampft, Ausbeute 12,9 g (73,9 % d. Th.) n_{D}²³ 1.517, viskoses Oel.

### Stufe 6: Herstellung von N-Chloracetyl-N-(1-methyl-2-methoxyethyl)-2-methyl-4-(4'-hydroxycarbonylbutoxy)-6-ethylanilin

9,9 g (30,6 mMol) des nach Stufe 5 hergestellten N-(1-Methyl-2-methoxyethyl)-2-methyl-4-(4'-hydroxycarbonylbutoxy)-6-ethylanilins werden in 400 ml Methylenchlorid gelöst und unter Rühren bei Raumtemperatur mit 30,6 ml (30,6 mMol) 1M Natronlauge versetzt. Dann lässt man 2,7 ml (33,67 mMol) Chloressigsäurechlorid bei Temperaturen zwischen 20° und 25°C langsam unter Rühren zutropfen. Das Reaktionsgemisch wird noch ca. 2 Stunden weitergerührt. Anschliessend werden nochmals unter den vorgenannten Bedingungen 10,2 ml 1M Natronlauge und danach 0,9 ml Chloressigsäurechlorid zugegeben und das Gemisch noch ca. 12 Stunden bei Raumtemperatur weitergerührt. Das resultierende Gemisch wird basisch eingestellt, mit Diethylether extrahiert, die Etherphase verworfen und die wässrige Phase mit Salzsäure auf pH 3 gebracht. Die wässrige Phase wird erneut mit Diethylether extrahiert, mit Eiswasser und gesättigter Kochsalzlösung mehrfach gewaschen, über Natriumsulfat getrocknet und eingedampft. Ausbeute 7,2 g (59 % d. Th.) viskoses Oel, n_{D}²³ 1.515. Die Struktur von Verbindung 6 wird mit Hilfe massenspektrometrischer Verfahren [Finnigan 4500 mit 'direct-exposure probe' (DEP)] bestätigt.

### Schematische Datstellung der Herstellung des zur Kupplung andas Trägergroteinbefähigten N-Chloracetyl-N-(1-methyl-2-methoxyethyl)-2-methyl-4-(4'-hydroxycarbonylbutoxy)-6-ethylanilins

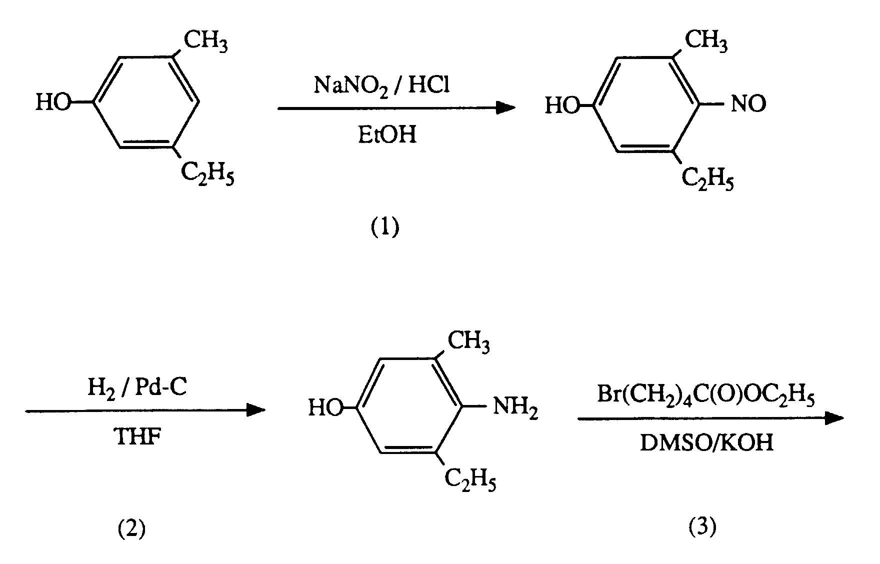

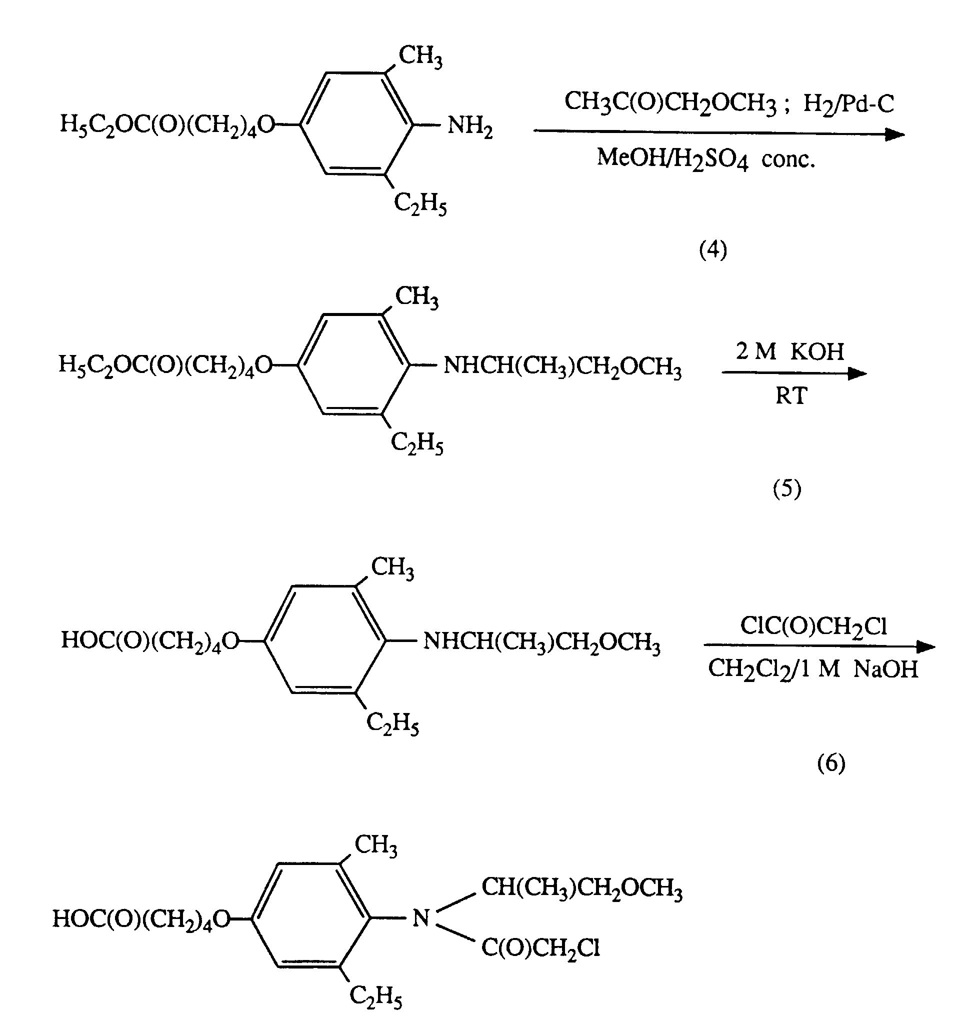

### 1.2: Metolachlor-Protein Konjugat

Das gemäss Beispiel 1 hergestellte Metolachlorderivat wird entweder an "Bovine Serum Albumine" (BSA; Fluka) oder an "Keyhole Limpet Hemocyanin" (KLH; Calbiochem) konjugiert unter Anwendung der aktivierten Ester-Methode (Kulkarni *et al*., 1981).

Im einzelnen wird dabei die Carboxylgruppe des Derivats in N,N-Dimethylformamid (DMF) (8 mg/200 µl) bei Raumtemperatur solubilisiert und anschliessend mit einem 4 molaren Ueberschuss von α-Hydroxysuccinimid (9.1 mg/200 µl DMF) sowie N,N'-Dicyclohexylcarbodiimid (16 mg/200 µl DMF) versetzt.
Der Reaktionsansatz wird zunächst 1 Stunde bei 22°C und anschliessend 18 Stunden bei 4°C gerührt.

Das bei der Reaktion gebildete weisse Präzipitat wird durch 3 minütige Zentrifugation mit 12'000 g bei Raumtemperatur entfernt und der aktivierte Ester anschliessend zu BSA oder KLH (24 mg) gegeben, das zuvor in 5.4 ml einer Phosphat-gepufferten Salzlösung [PBS-Puffer, 0.01 M Natriumphosphat und 0.145 M NaCl, pH 7.0] solubilisiert worden ist. Das molare Verhältnis von [Derivat]/[BSA] beträgt dabei ca. 55/1 (8 mg Derivat/24 mg BSA).

Nach 4stündiger Inkubation bei einer Temperatur von 4°C wird das gebildete Präzipitat durch 10 minütige Zentrifugation mit 2'000 g bei 4°C entfernt und der verbleibende Ueberstand mit dem Proteinkonjugat ausgiebig gegen PBS [enthaltend 3 mM NaN₃] dialysiert, bevor er dann für die Immunisierungs-Experimente verwendet wird.

Das Ausmass der Kupplungsreaktion wird mit Hilfe einer SDS-Gelelektrophorese sowie durch Absorptionsspektrophotometrie bei 280 nm bestimmt, was sowohl dem UV Peak des BSA als auch dem der Verbindung 6 entspricht [Aₘₐₓ= 274 nm]. Das molare Verhältnis von Metolachlor zu BSA liegt bei ca. 23: 1.

### 1.3: Konjugation des Haptens an alkalische Phosphatase

16 µg des gemäss Beispiel 1.2 hergestellten aktiven Esters des N-Chloracetyl-N-(1-methyl-2-methoxyethyl)-2-methyl-4-(4'-hydroxycarbonylbutoxy)-6-ethylanilin wird zu 40 µl alkalischer Phosphatase aus Kälberdarm (153 µg/230 U) [Calbiochem, EIA grade] zugegeben. Nach 4 stündiger Inkubation bei 4°C wird das Reaktionsgemisch ausgiebig gegen PBS, enthaltend 3mM NaN₃, dialysiert. Die enzymatische Aktivität des Konjugats wird mit Hilfe von p-Nitrophenylphosphat als Substrat bestimmt. Die Aktivität des Enzyms bleibt nach der Hapten-Konjugation unverändert.

### Beispiel 2: Immunisierung

Gruppen von jeweils 5 weiblichen BALB/c Mäusen (Tierfarm Sisseln, Schweiz), die zwischen 4 und 6 Wochen alt sind, erhalten 3 Serien von intraperitonealen bzw. subkutanen Injektionen mit KLH-konjugiertem Metolachlor (50 µg/Injektion).

Die erste Injektion beinhaltet 0,1 ml des Konjugates in PBS, das in einem Verhältnis von 1:1 mit 0,1 ml komplettem Freund Adjuvanz vermischt ist.

50 µl dieser Injektionslösung werden intraperitoneal injiziert, die restlichen 150 µl subcutan.

Bei der zweiten und dritten Injektionsserie, die 14 bzw. 30 Tage nach der Erstapplikation erfolgt, wird das komplette Freund Adjuvans durch inkomplettes ersetzt.

1 Woche nach der letzten Injektion wird Blutserum von den Versuchstieren entnommen und die Bluttiter mit Hilfe eines ELISA-Tests bestimmt, wobei die Mikrotiterplatten zuvor mit BSA-konjugiertem Hapten beschichtet werden (siehe Abschnitt 6).

Nach einer Ruheperiode von 2 Monaten erfolgt eine weitere einmalige intraperitoneale Injektion des KLH-Konjugates in einer Dosierung von 370 µg/200 µl PBS.

### Beispiel 3: Fusionsprotokoll

### 3.1. Gewinnung von "Feeder"-Zellen (peritoneale Makrophapen).

Unbehandelte, ca. 6 bis 8 Wochen alte Balb/c-Mäuse werden einen Tag vor der beabsichtigten Fusion getötet und durch Eintauchen in 70 %igem Alkohol sterilisiert.

Anschliessend wird das Fell und die obere Bauchhaut steril angeschnitten ohne das Peritoneum zu verletzen. Mit einer sterilen 5 ml Plastikspritze und einer sterilen 18-er Injektionsnadel werden 4 ml BSS (ohne Ca²⁺ und Mg²⁺) und 1 ml Luft in die Bauchhöhle injiziert.

Nach leichtem Massieren des Bauches (wobei Spritze und Nadel in der Bauchhöhle verbleiben) wird der zuvor injizierte BSS-Puffer wieder aus dem Peritoneum abgezogen und in ein steriles Falconröhrchen gegeben. Dieses Procedere wird noch 2 mal wiederholt. Die so gewonnenen Makrophagen werden mit Eis gekühlt und anschliessend 2 x mit je 20 ml BSS gewaschen.

Die Makrophagen werden dabei je 10 Min. bei 300 g und einer Temperatur von 5°C zentrifugiert. Das Pellet wird dann in 50 ml HAT-Medium resuspendiert und die Zellsuspension auf 4 Costarplatten mit insgesamt 24 Vertiefungen verteilt (0,5 ml/Vertiefung).

Anschliessend werden die so vorbereiteten Makrophagen bei einer Temperatur von 37°C und einer CO₂-Konzentration von 6 % in einem Inkubator aufbewahrt.

Pro Fusionsvorgang werden ca. 4 x 10⁶ Makrophagen benötigt.

### 3.2. Anzucht der Myeloma-Zellinie Sp 2/0-Ag 14

Bei der genannten Myeloma-Zellinie Sp 2/O-Ag 14 handelt es sich um eine Myeloma-Zellinie, die selbst keine Antikörper sezerniert und die bei M. Shulman *et al*. (1978) beschrieben ist. Diese Myeloma-Zellinie kann bei der "American Type Culture Collection" in Rockville, Maryland bezogen werden.

Pro Fusion benötigt man 50 ml einer gut gewachsenen Kultur, die mindestens 10 Millionen Zellen enthält. Die Kultivierung der Myelomzellen erfolgt vorzugsweise in T 175 "Falcon"-Flaschen (Fa. Beckton & Dickenson).

Einen Tag vor der Fusion wird das Kultivierungsmedium (RPMI 1640) durch frisches RPMI 1640-Medium ersetzt. Am Tag der Fusion werden die Sp 2/0-Ag 14 Zellen geerntet, in ein steriles 50 ml Plastikröhrchen gegeben und 10 Min. bei 300 g und einer Temperatur von 5°C zentrifugiert (MSE Zentrifuge, Modell Chilspin, UK). Nach der Zentrifugation wird der Ueberstand abgezogen und verworfen. Die Zellen werden 2 x mit je ca. 30 ml BSS-Puffer (Ca²⁺ und Mg²⁺ frei) gewaschen (10 Min. bei 300 g, 5°C) und anschliessend in 5 ml BSS resuspendiert.

Ein Aliquot der Zellsuspension wird zur Bestimmung der Zellzahl entnommen und mit Fluoresceindiacetat (FDA) gefärbt. Bis zur Weiterverwendung werden die Myelomzellen auf Eis aufbewahrt.

### 3.3. Herstellung einer Milzzellsuspension

Die Entnahme der Milz aus einer zuvor gemäss Beispiel 2 immunisierten Balb/c-Maus erfolgt unter sterilen Bedingungen und unter Kühlung mit Eis.

Die zuvor immunisierte Balb/c-Maus wird durch Genickbruch getötet und die Milz steril entnommen. Dazu wird die Maus kurz in 70 %igen Ethanol eingetaucht und mit sterilem Besteck präpariert. Die Milz wird vorsichtig entnommen und auf ein feines Nylonnetz gelegt. Dort wird sie mit einer Schere fein zerschnitten und dann mit Hilfe eines 5 ml Spritzenstempels vorsichtig durch das Netz gedrückt, ohne dabei zu viele Zellen zu zerstören. Während des ganzen Vorgangs wird das Netz mit BSS gespült.

Die so gewonnene Zellsuspension wird in 50 ml Plastikröhrchen gegeben und 10 Min. bei 300 g und einer Temperatur von 5°C zentrifugiert (MSE Zentrifuge, Modell Chilspin; UK). Die Zellen werden anschliessend 2 x mit je 20 ml BSS gewaschen (10 Min.; 300 g; 5°C; MES Chilspin) und das Zellpellet wird nach der Zentrifugation in 10 ml BSS resuspendiert.

Bis zur Fusion mit Sp 2/O-Ag14 Myelomzellen werden die Milzzellen auf Eis belassen.

### 3.4. Fusion: Milzzellen und Sp 2/O-Ap 14 Myelomzellen

Das Verhältnis von Myelomzellen zu Milzzellen sollte für die Fusion 1:10 betragen.

Milzzellen (in BSS-Puffer) und Sp 2/O-Ag14 Myelomzellen (in BSS-Puffer) werden in dem angegebenen Verhältnis zusammengegeben und 10 Min. bei 300 g und einer Temperatur von 5°C zentrifugiert (MSE Zentrifuge, Modell Chilspin). Das Pellet wird nochmals in BSS-Puffer resuspendiert und die Suspension anschliessend erneut zentrifugiert. Das Pellet wird vorsichtig aufgerührt und in 37°C warmes Wasserbad gestellt. Es wird dann 1 ml vorgewärmtes und steriles PEG-4000 (MERCK) über einen Zeitraum von 60 Sek. tropfenweise zu den Zellen zugegeben, wobei der gesamte Ansatz ständig bewegt wird. Die Zellen werden anschliessend noch 30 Sek. lang weitergeschüttelt, bevor 5 ml einer zuvor erwärmten BSS-Puffers (ohne Ca²⁺, Mg²⁺) ebenfalls tropfenweise über einen Zeitraum von ca. 5 Min. unter ständigem Rühren dazugegeben werden.

Die auf die beschriebene Weise fusionierten Zellen werden dann abzentrifugiert (10 Min.; 300 g; 20°C, MSE Zentrifuge, Modell Chilspin), der Ueberstand wird abgezogen und verworfen. Das Zellpellet wird in 50 ml HAT-Medium resuspendiert und die so erhaltene Zellsuspension auf die vorbereiteten 4 Costarplatten (Mikrotiterplatten mit 24 Vertiefungen, Durchmesser pro Vertiefung 24 mm; Gesamtfläche für Zellwachstum 2,0 cm²) verteilt (0,5 ml/Vertiefung).

Die Inkubation der Costarplatten erfolgt bei einer Temperatur von 37°C und bei einer CO₂-Konzentration von 6 %.

### Beispiel 4: Kultivierung der Hybridzellen

Am 1. Tag nach der Zellfusion wird 1 ml HAT-Medium pro Vertiefung zu den Kulturplatten zugegeben. 3 bis 4 Tage nach der Zellfusion erfolgt eine mikroskopische Kontrolle der fusionierten Zellen. Gleichzeitig wird das verbrauchte Medium abgesaugt und durch 1 ml frisches HAT-Medium ersetzt. Nach weiteren 3 Tagen (6 - 7 Tage nach der Zellfusion) erfolgt ein erneuter Wechsel des Kulturmediums. Ab dem 7. bis 10. Tag nach der Zellfusion wird jede Vertiefung mikroskopisch nach Hybriden abgesucht und 2 bis 3x wöchentlich das Medium erneuert.

Sobald in einer Vertiefung Hybride gewachsen sind, kann das HAT Medium dort durch HT-Medium ersetzt werden. Der Ueberstand von gewaschenen Hybridkulturen (mindestens 10 % der Vertiefungen) wird mit einer sterilen Pasteurpipette abgehoben und auf das Vorhandensein von Antikörpern getestet.

Sobald die Vertiefungen mit positiven Hybridkolonien voll bewachsen sind, können diese auf neue Costarplatten in RPMI 1640-Medium transferriert werden, wobei der Inhalt einer vollbewachsenen Vertiefung auf 2 bis 3 neue Vertiefungen verteilt wird.

### Beispiel 5: Klonierung der positiven Hybridzellen

Die Zellen einer positiven Vertiefung werden mit Hilfe einer Pipette gelöst und in 1 ml Medium in ein Röhrchen überführt. Anschliessend wird ein Aliquot zur Bestimmung der Zellzahl entnommen und mit FDA angefärbt (Verdünnung 2 mit FDA: 50 µl 1 : Zellen + 50 µl Farbstoff). Die bevorzugte Zellzahl liegt bei 10⁵ bis 10⁶ Zellen/ml. Anschliessend werden die Hybridzellen in einem Verhältnis von 1 : 100 mit HT-Medium verdünnt (z.B. 100 µl Zellen + 9,9 ml HT-Medium).

In zwei 50 ml Falconröhrchen werden je 25 ml HT-Medium vorgelegt und mit 5 ml einer Macrophagensuspension auf insgesamt 30 ml pro Röhrchen aufgefüllt. Die Macrophagen werden zuvor aus einer Maus isoliert und in 10 ml HT-Medium resuspendiert (vgl. Abschnitt 3.1 ).

In diesen die Macrophagen enthaltenden Falcon-Röhrchen werden die Hybridzellen verdünnt, bis eine Zelldichte von (i) 270 Zellen/30 ml bzw. (ii) 90 Zellen/30 ml erreicht ist. Anschliessend werden diese Ansätze auf Costarplatten (Mikrotiterplatten mit 96 Vertiefungen) verteilt, wobei je 200 µl pro Vertiefung zugegeben werden. Dies entspricht einer Zellzahl von (i) 1,8 Zellen/Vertiefung bzw. (ii) 0,6 Zellen/Vertiefung. Pro Verdünnung benötigt man auf diese Weise 1,5 Mikrotiterplatten.

Nach 7 Tagen werden die einzelnen Vertiefungen mikroskopisch kontrolliert und die Vertiefungen die Zellklone enthalten erfasst. Diejenige Verdünnung, bei der ca. 50 % der Vertiefungen Zellklone enthalten wird für den ELISA-Test verwendet. Dies sollte in der Regel die Verdünnung mit 0,6 Zellen/Vertiefung sein.

Nach ca. 7 - 10 Tagen werden die Ueberstände der positiven Vertiefungen (mit Klonen) in einem ELISA-Test auf die Anwesenheit von monoklonalen Antikörpern getestet und die positiven Klone auf Costarplatten (mit 24 Bohrungen) in RPMI 1640-Medium vermehrt. Aliquots dieser positiven Klone werden in flüssigem Stickstoff aufbewahrt.

### Beispiel 6: Hybridoma-Screening (ELISA-Test)

Zunächst werden 100 µl einer Lösung von BSA-konjugiertem Hapten in Natriumcarbonat-Puffer (50 mM, pH 9.6) in die einzelnen Vertiefungen einer Mikrotiterplatte gegeben und dieser Ansatz bei 4°C in einer feuchten Kammer über Nacht inkubiert. Anschliessend werden die Vertiefungen jeweils 5 x mit einem 0,1 % PBS-Tween Puffer gewaschen. Zur Blockierung der unbesetzten Bindungsstellen auf der Mikrotiterplatte werden 200 µl einer PBS-BSA Lösung (1 %) in jede Vertiefung gegeben. Dieser Ansatz wird 1 - 2 Stunden bei Raumtemperatur inkubiert und anschliessend mit einem 0,1 %igen PBS-Tween-Puffer gewaschen.

Man gibt dann zu jeder Vertiefung 200 µl des Hybridoma-Überstands, der im Verhältnis 1:2 mit PBS-Tween (0,1 %) verdünnt ist, hinzu und inkubiert den gesamten Ansatz für 2 Stunden bei Raumtemperatur. Anschliessend werden die Vertiefungen erneut 5 x mit einem 0,1 %igen PBS-Tween-Puffer gewaschen.

Es folgt die Inkubation mit Phosphatase-konjugiertem Ziegen anti-Maus Antikörper (Kirkegaard & Perry Lab.). Zunächst werden 100 µl eines über eine Affinitätschromatographie gereinigten Ziegen Antikörpers gegen Mäuse IgG, der 1 : 1500 in PBS-Tween (0,12 %) verdünnt vorliegt (Kirkegaard & Perry Laboratories) und mit alkalischer Phosphatase markiert ist, zu jeder Vertiefung hinzugegeben.

Die Inkubationszeit beträgt 1,5 Stunden bei Raumtemperatur. Anschliessend werden die einzelnen Vertiefungen erneut mit PBS-Tween (0,1 %) gewaschen (5 x).

Danach werden 150 µl einer substrathaltigen Lösung (1 mg/ml p-Nitrophenylphosphat) in jede Vertiefung zugegeben. Nach einer Inkubationszeit von 2 Stunden im Dunkeln erfolgt die spektroskopische Bestimmung bei 405 nm. Positive Hybridoma-Zellen, die einen spezifischen Antikörper sezernieren geben ein starkes positives Signal bei der gewählten Wellenlänge.

### Beispiel 7: Expansion der Hybridomazellen in der Maus

Für die Anregung der Ascites-Produktion werden weibliche Balb/c Mäuse (20 g-25 g) (Tierfarm Sisseln, CH) mit 0.3 ml Pristan Oel (Aldrich Chemical) vorbehandelt, das intraperitoneal injiziert wird. 1 bis 3 Wochen nach der Pristan Applikation erhalten die Mäuse eine zweite Injektion (0,2 ml Pristan-Oel, i.p.). Gleichzeitig mit dieser 2. Injektion erhalten die Tiere 2 x 10⁶ Hybridomazellen in 0,2 ml PBS.

Die aus dieser Behandlung resultierende Ascitesflüssigkeit wird gesammelt, bei 800 g zentrifugiert und bei einer Temperatur von -20°C aufbewahrt. Nach dem Auftauen wird die Ascitesflüssigkeit 1 Stunde bei 30'000 g zentrifugiert. Die oberste Schicht, die vorwiegend Lipide enthält, wird entfernt. Anschliessend wird die Proteinkonzentration bestimmt und auf einen Wert von 10 mg/ml eingestellt durch Zugabe von PBS.

Die Immunglobulin G Fraktion (IgG) wird durch tropfenweise Zugabe von 0,9 Volumenteilen einer gesättigten Ammoniumsulfatlösung bei 0°C präzipitiert. Nach 1 Stunde wird die IgG-Fraktion pelletiert durch einstündige Zentrifugation bei 22'000 g. Das Pellet wird anschliessend in 20 nM-Tris-HCl Puffer, pH 7,9, der 50 mM NaCl enthält, aufgelöst und gegen den gleichen Puffer über Nacht bei 4°C dialysiert.

Die weitere Aufarbeitung der IgG Fraktion geschieht mit Hilfe einer Anionen-Austauschchromatographie an einer DE-52 Diethylaminoethylcellulose (Whatman) Säule. Die Probe wird 1:2 (v/v) mit 20 mM Tris-HCl, pH 7,9 verdünnt, bis eine NaCl Endkonzentration von 25 mM erreicht ist und 10 mg Protein/ml Gel werden auf die Säule aufgetragen. Die Elution wird erreicht durch Erhöhung der NaCl-Konzentration von 25 mM auf 200 mM (linearer Gradient). Im allgemeinen erfolgt die Elution monoklonaler Antikörper im Bereich von 80 mM NaCl.

Die Fraktionen werden über Nacht bei einer Temperatur von 4°C gegen PBS dialysiert und bei -70°C aufbewahrt. Der Reinheitsgrad wird mit Hilfe einer Natriumdodecylsulphat Polyacrylamid-Gelelectrophorese (SDS-PAGE) bestimmt, sowie durch isoelektrische Fokussierung.

Im vorliegenden Fall liegt der Reinheitsgrad bei >90 %.

### Beispiel 8: Metolachlor-Nachweis

### 8.1: indirekter ELISA (Assay A)

Der Nachweis von Metolachlor erfolgt mit Hilfe eines zweistufigen, kompetitiven ELISA-Tests unter Verwendung eines Enzym-markierten zweiten Antikörpers.

BSA-konjugiertes Hapten wird zunächst in einem 50 mM Natriumcarbonatpuffer (pH 9,6) (200 ng BSA-konjugiertes Hapten/100 µl Natriumcarbonat-Puffer) an Mikrotiterplatten (z.B. Dynatech, Typ M 129A) adsorbiert und über Nacht bei einer Temperatur von 4°C inkubiert. Es hat sich gezeigt, dass durch Reduktion der Konjugat[BSA/Hapten]-Konzentration von 200 ng/100 µl Natriumcarbonat-Puffer auf 50 ng/100 µl Natrium-carbonat-Puffer die Empfindlichkeit des Assay aufgrund eines verbesserten Coatings um fast das 5fache gesteigert werden kann.

Die Platten werden anschliessend 5 x mit PBS-Puffer gewaschen, der mit 0,1 % (v/v) Polysorbat 20 (Tween 20) angereichert ist (PBS-Tween).

Die restlichen freien Bindungsstellen des festen Trägermaterials werden dann durch Zugabe von BSA in Form einer 1 % Lösung blockiert. Nach zweistündiger Inkubation bei 22° C werden die Platten erneut mit PBS-Tween (0.1 %) gewaschen.

50 µl der zuvor gereinigten monoklonalen Antikörper (0.2 µg/ml) oder aber des Ueberstandes der Zellklone (in einer Verdünnung von 1:15) werden a) mit 950 µl einer Standardlösung, die einen steigenden Anteil an Metolachlor bzw. Metolachlor-Analogen enthält, b) mit Metolachlor-haltigen Wasserproben oder c) Metolachlor-haltigen Bodenextrakten inkubiert. (Alle Verdünnungen werden in PBS-Tween vorgenommen).

Nach einer Inkubationszeit von 1 Stunde bei Raumtemperatur (22° C) werden 200 µl des Antigen/Antikörper Gemisches zu jeder Vertiefung der Mikrotiterplatte zugegeben und der gesamte Ansatz wird für eine weitere Stunde inkubiert. Die Vertiefungen werden anschliessend 5 x mit PBS-Tween (0.1 %) gewaschen und mit 100 µl/Vertiefung Ziegen-anti-Maus IgG Antikörper, der konjugiert an alkalische Phosphatase vorliegt (Verdünnung 1:1500), beschickt und für einen Zeitraum von 1,5 Stunden inkubiert.

Nach erneutem Waschen werden 150 µl/Vertiefung des in 1 mg/ml Diethanolaminpuffer (1 mM, pH 9,8, angereichert mit 0,5 mM MgCl₂ x 6H₂O) gelösten Substrates p-Nitrophenylphosphat zu den Vertiefungen zugegeben.

Nach einer Inkubationszeit von 2 Stunden bei einer Temperatur von 22° C lässt sich ein Farbumschlag beobachten, der proportional ist zur Menge an Antikörper, der mit dem an die Festphase gebundenen Antigen reagiert hat. Die Intensität der eingetretenen Farbreaktion wird bei einer Wellenlänge von 405 nm bestimmt. Die Verdünnungen der einzelnen Proben werden so gewählt, dass man ohne Zugabe eines Inhibitors (Bo) Absorptionswerte in einem Bereich zwischen 0.3 und 0.5 erhält. Für die Kontrollen (ohne Antikörper und mit nicht nachweisbaren Mengen an Antigen) ergeben sich Werte von R ≦ 0.005. Alle Proben werden in dreifacher Ausfertigung bestimmt.

### 8.2: direkter ELISA (Assay B)

Mikrotiterplatten [Dynatech M 129A] werden mit monoklonalem Antikörper [z.B. MAb 4082-25-4; 75 ng/100 µl 50 mM Natriumcarbonat Puffer, pH 9.6] beschichtet und über Nacht bei einer Temperatur von 4°C inkubiert.
Die Platten werden dann 5 mal mit 0.1 % (v/v) PBS-Tween gewaschen und anschliessend für 2 Stunden mit PBS, das mit 1% (w/v) BSA angereichert ist, inkubiert um die noch freien Bindungsstellen zu blockieren. Nach erneutem Waschen werden jeweils 150 µl einer Standardlösung in die Vertiefungen der Mikrotiterplatten gegeben, die steigende Mengen an Metolachlor in 0.1 % (v/v) PBS-Tween enthalten. Es folgt eine 1 stündige Inkubation bei einer Temperatur von 22°C.
Anschliessend werden pro Vertiefung 50 µl des Hapten-Enzym [alkalische Phosphatase]-Konjugates [2 µg/ml] zugegeben und die Inkubation wird für eine weitere Stunde fortgesetzt. Nach erneutem Waschen wird in jede Vertiefung 150 µl des p-Nitrophenyl-phosphat Substrates in einer Konzentration von 1 mg/ml in Diethanolamin Puffer [1 mM, pH 9.8, angereichert mit 0.5 mM MgCl₂• 6H₂O] zugegeben. Nach 2 stündiger Inkubation bei 22°C im Dunkeln wird die Absorption bei 405 nm bestimmt.

### 8.3: Bestimmung des Metolachlorgehaltes

Zur Ermittlung der in einer Probe enthaltenen Menge an Metolachlor wird zunächst eine Eichkurve erstellt (Abb. 1), wobei B/Bo x 100 gegen die Konzentration an Inhibitor aufgetragen wird. (Bo bedeutet Absorptionsfähigkeit gemessen ohne Zugabe eines Metolachlorininhibitors zum Antikörper, und B Absorptionsfähigkeit bei Zugabe verschieden konzentrierter Metolachlor-Inhibitoren). Der I₅₀-Wert gibt diejenige Konzentration des Antigens an, bei der die Antikörperbindung an die Festphase zu 50 % gehemmt wird. Der I₅₀-Wert wird mit Hilfe eines auf die vorliegenden Verhältnisse speziell angepassten ENZFITTER (Leatherbarrow, Elsevier-Biosoft) Kurvenkalkulationsprogramms bestimmt, basierend auf einer 4 Parameter umfassenden logistischen Kurve (Raab GM, 1983). Auch die quantitative Metolachlorbestimmung in Boden- oder Wasserproben im Rahmen des ELISA wird mit Hilfe des ENZFITTER-Programms durchgeführt, wobei die Anpassung der Kurve auf Standards basiert, die auf jeder Mikrotiterplatte mitlaufen.

### Beispiel 9: Analyse von Bodenproben

Aliquots (2 g) standardisierter Bodenproben unterschiedlicher Herkunft werden in einem Extraktor 4 Stunden mit 20 ml eines Methanol/Wasser [80/20 (v/v)] Gemisches extrahiert. Für den kompetitiven ELISA werden die Bodenextrakte gewöhnlich in einem Verhältnis von 1:40 in PBS-Tween (0.1 %) verdünnt, um einer möglichen Denaturierung der monoklonalen Antikörper durch das Methanol vorzubeugen. Anschliessend werden die verdünnten Bodenproben (950 µl) auf die zuvor beschriebene Weise entweder mit dem spezifischen anti-Metolachlor Antikörper [MAb 4082-85-4] (50 µl) vermischt und anschliessend mit dem trägergebundenen Hapten inkubiert [indirekter ELISA] oder aber direkt mit den trägergebundenen anti-Metolachlor Antikörpern zur Reaktion gebracht und dann mit Enzym-markiertem Antigen inkubiert [direkter ELISA].

### Beispiel 10: Analyse von Wasserproben

Für den kompetitiven ELISA werden 100 µl eines 10fach konzentrierten PBS-Tween Puffers zu 850 µl einer Wasserprobe zugegeben. Die Metolachlorbestimmung kann dann analog der zuvor beschriebenen Vorgehensweise durchgeführt werden [vergl. Beispiel 9].

### II. ERGEBNISSE

### a) Herstellung monokonaler Antikörper

Insgesamt werden 24 Zellfusionen zwischen Myelomazellen und Milzzellen, die zuvor aus einer mit einem KLH-Metolachlor immunisierten BALB/c Maus isoliert wurden, durchgeführt. Die Fusionseffizienz liegt bei ca. 90 %. 7 der auf diese Weise erhältlichen Zellkolonien produzieren monoklonale Antikörper, welche im ELISA-Test eine sehr starke Reaktion zeigen. Diese werden mit Hilfe der limitierenden Verdünnungsmethode (vgl. Abschnitt 5) kloniert.

Auf diese Weise konnten 7 monoklonale Antikörper erhalten werden, von denen einer [MAb 4082-25-4; (IgGl Subklasse)] eine sehr hohe Affinität gegenüber Metolachlor zeigt, während die Affinität der übrigen 6 monoklonalen Antikörper auf das Metolachlorderivat beschränkt bleibt.
Dieser Antikörper wurde dazu verwendet zwei kompetitive Immunoassays zu entwickeln. Einer dieser Assays basiert auf der Verwendung von Mikrotiterplatten, die mit BSA-Hapten-Konjugat beschichtet sind sowie von Enzym-markierten Ziegen/anti-Maus Antikörpern [indirekter ELISA].
Der zweite Assay verwendet Hapten-Enzym-Konjugate sowie Antikörper-beschichtete Mikrotiterplatten [direkter ELISA].

Beide Assays sind in der Lage Metolachlor im ppb Bereich nachzuweisen. Der direkte ELISA ist geringfügig weniger empfindlich als der indirekte, dafür aber um ein Vielfaches schneller. Die I₅₀- Werte liegen bei 1.0 ng/ml für den direkten bzw bei 0.6 ng/ml für den indirekten ELISA. Der Nachweisbereich für Metolachlor in Puffer liegt zwischen 0.1 ng/ml und 10 ng/ml, wobei die minimale Nachweisgrenze als diejenige Konzentration definiert ist, die man benötigt um eine prozentuale Erhöhung der gebundenen Antikörper zu erreichen, die der zweifachen Standardabweichung des Blindwertes entspricht. Der Variationskoeffizient für Wiederholungsbestimmungen im 1 ppb Bereich beträgt 11% (20 Bestimmungen).

Die Kreuzreaktivität von MAb 4082-25-4 kann mit Hilfe eines indirekten kompetitiven ELISA bestimmt werden. Wie die Tabelle 1 zeigt findet man eine schwache Kreuzreaktivität von 1.4 % mit dem strukturell sehr nahe liegenden Hydroxy-Metaboliten [Verbindung A] des Metolachlor. Die übrigen Metaboliten zeigen keine Kreuzreaktivitäten [< 0.1 %]. Der monoklonale Antikörper bindet nicht an andere Chloracetanilid-Herbizide und verwandte Verbindunden wie z.B. Alachlor, Furalaxyl und Matalaxyl [< 0.1 %].

### Hinterlegung:

Die im Rahmen der vorliegenden Erfindung hergestellten und verwendeten Hybridoma-Zellinien [4082-25-4] wurden bei der als Internationale Hinterlegungsstelle anerkannten'European Collection of Animal Cell Cultures'(ECACC) in Salisbury, UK, entsprechend den Anforderungen des Budapester Vertrages für die Internationale Anerkennung der Hinterlegung von Mikroorganismen zum Zwecke der Patentierung, unter der Hinterlegungsnummer ECACC 9002 1701 hinterlegt. Eine Erklärung zur Lebensfähigkeit der hinterlegten Proben wird durch die besagte Internationale Hinterlegungsstelle ausgefertigt.

### III MEDIEN UND PUFFER

### (A) RPMI 1640-Medium

RPMI 1640 (Seromed) mit folgenden Zusätzen:

| | |
|---|---|
| Kälberserum | 15 % |
| L-Glutamin | 4 mM |
| Gentamicin | 0.01% |
| Natrium-Pyruvat | 1 mM |
| 2-Mercaptoethanol | 50 µM |
| Insulin | 5 µM |
| Transferrin | 5 µM |
| Selen (ITS) | 5 µM |

### (B) HAT-Medium

1 Liter RPMI 1640 Medium mit 20 ml Zusatz von HAT conc. (50 x) von Boehringer, das die folgende Zusammensetzung aufweist:

| | |
|---|---|
| Hypoxanthin 680 | 5.0 mg/l |
| Aminopterin | 8.8 mg/l |
| Thymidin | 193.8 mg/l |

### (C) HT-Medium

1 Liter RPMI 1640 Medium mit 20 ml Zusatz von HT conc. (50 x) von Boehringer, das die folgende Zusammensetzung aufweist:

| | |
|---|---|
| Hypoxanthin 680 | 5.0 mg/l |
| Thymidin | 193.8 mg/l |

### (D) BSS-Puffer [Earle's Salzlösung, ohne Ca und Mg, pH 7.4]

| | |
|---|---|
| KCl | 7.3 mM |
| NaCl | 116.0 mM |
| NaHCO₃ | 26.0 mM |
| NaH₂PO₄• 2H₂O | 1.0 mM |
| Glucose | 5.5 mM |
| Phenolrot | 48.0 µM |

1% Zusatz (v/v) einer Penicillin/Streptomycin-Lösung (Seromed) [10'000 E Penicillin, 10 mg/ml Streptomycin]

### (E) Natriumcarbonat-Puffer [pH 9.6]

| | |
|---|---|
| Na₂CO₃ | 477.0 mg |
| NaHCO₃ | 879.0 mg |
| NaN₃ | 1.8 mg |
| ad 300 ml H₂O | |

### (F) PBS-Puffer [pH 7.0]

| | |
|---|---|
| NaCl | 8.5 g |
| Na₂HPO₄• 2H₂O | 1.28 g |
| NaH₂PO₄• 2H₂O | 0.436 |
| ad 1000 ml H₂O | |

### (G) PBS-TWEEN-20 [0.1%]

1 ml Tween-20 (Serva) + 1000 ml PBS

### (H) PBS-BSA [1%]

| | |
|---|---|
| BSA | 5.0 g |
| NaN₃ (0.5 M) | 3.0 ml |
| ad 500 ml PBS | |

### (I) Substrat-Puffer [Diethanolamin-Puffer, pH 9.8]

| | |
|---|---|
| Diethanolamin | 97.0 ml |
| NaN₃ (0.5 M) | 6.0 ml |
| MgCl₂• 6H₂O | 100.0 mg |
| ad 1000 ml H₂O, Einstellen des pH-Wertes auf pH 9.8 mit HCl conc. | |

Herstellung des Substrates: Unmittelbar vor Gebrauch wird eine Substrattablette (= 5 mg) des p-Nitrophenylphosphat-Substrates (Sigma 104) in 5 ml Substratpuffer gelöst.

**Tabelle 1:**

| Kreuzreaktivität der bekanntesten Metolachlor-Analogen mit MAb 4082-25-4 [Die Metolachloranalogen A bis N sind in Tabelle 2 anhand ihrer Formel wiedergegeben]. | | |
|---|---|---|
| Verbindung | I₅₀ [ng/ml]* | Kreuzreaktivität [%]^{#} |
| Metolachlor | 0.6 | 100 |
| Alachlor | > 1000 | < 0.1 |
| A | 43 | 1.4 |
| B | > 1000 | < 0.1 |
| C | > 1000 | < 0.1 |
| D | > 1000 | < 0.1 |
| E | > 1000 | < 0.1 |
| F | > 1000 | < 0.1 |
| G | > 1000 | < 0.1 |
| H | > 1000 | < 0.1 |
| I | > 1000 | < 0.1 |
| J | > 1000 | < 0.1 |
| K | > 1000 | < 0.1 |
| L | > 1000 | < 0.1 |
| M | > 1000 | < 0.1 |
| N | > 1000 | < 0.1 |

| | | |
|---|---|---|
| *I₅₀ = Inhibitorkonzentration für eine 50%ige Hemmung im kompetitiven ELISA. | | |
| #Kreuzreaktivität = Metolachlorkonzentration für 50% Hemmung / Konzentration des Metolachloranalogen für 50% Hemmung x 100. | | |

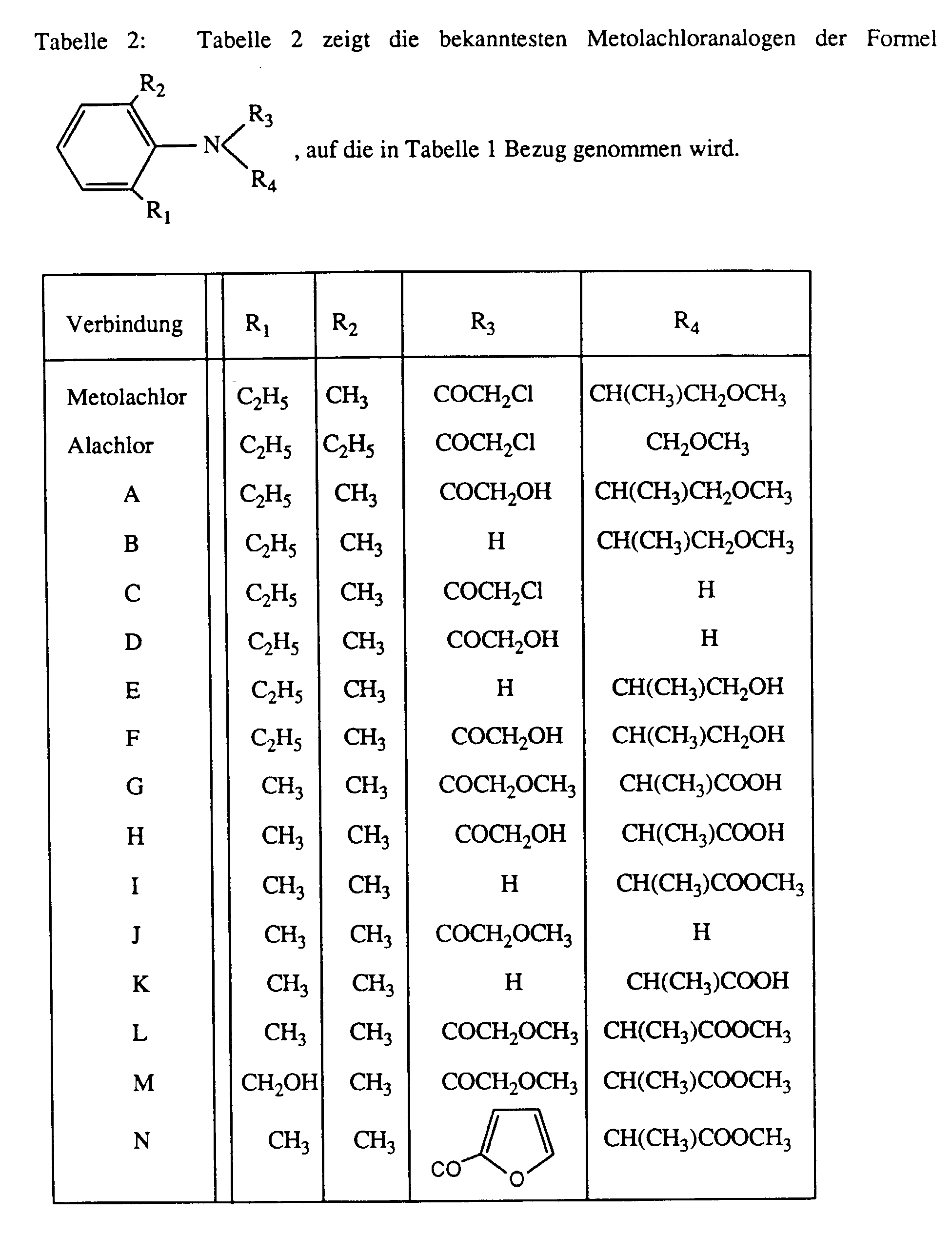

### IV LITERATUR

Ercegovich CD et al, J. Agric. Food Chem., 29: 559-563, 1981
Feng *et al*, J. Agric. Food Chem., 38: 159-163, 1990
Fleeker J, J. Assoc. Off. Anal. Chem., 70: 874-878, 1987
Hargrave HS und Merkle MG, Weed Sci., 19: 1971
Kawamura H, Berzojsky JA, J. Immunol., 136: 58, 1986 ,
Kelley M et al, J. Agric. Food Chem., 33: 962-965, 1985
Köhler G, Milstein, Nature, 256: 495-497, 1975
Kulkarni NP et al, Cancer Res., 41: 2700-2706, 1981
Littlefield JW, Science, 145: 709, 1964
Newsome WH, J. Agric. Food Chem., 33: 528-530, 1985
Raab GM, Clin. Chem., 29: 1757-1761, 1983
Schlaeppi J-M *et al*, J. Agric. Food Chem., 37: 1532-1538, 1989
Shulman M et al, Nature, 276: 269-270, 1978
van Rensburg E, Analyst, 110: 733., 1985
Wie SI, Hammock BD, J. Agric. Food Chem., 30: 949-957, 1982

### Patentliteratur

US-P 4,530,786

## Patentansprüche

1. Eine Hybridomazellinie, die einen für Metolachlor spezifischen monoklonalen Antikörper produziert, dadurch gekennzeichnet, dass die Kreuzreaktivitäten des Antikörpers mit den in Tabelle 1 angegebenen, strukturell verwandten Metolachlor-analogen < 2% beträgt.

2. Eine Hybridomazellinie gemäss Anspruch 1, die einen monoklonalen Antikörper produziert, der das in Tabelle 1 wiedergegebene Kreuzreaktivitätsmuster aufweist.

3. Eine Hybridomazellinie gemäss Anspruch 1, welche die kennzeichnenden Charakteristika von ECACC 9002 1701 besitzt.

4. Ein für Metolachlor spezifischer monoklonaler Antikörper und Derivate davon, die eine Kreuzreaktivität mit den in Tabelle 1 angegebenen, strukturell verwandten Metolachloranalogen von < 2% zeigen.

5. Monoklonaler Antikörper und Derivate davon gemäss Anspruch 4, die das in Tabelle 1 wiedergegebene Kreuzreaktivitätsmuster aufweisen.

6. Monoklonaler Antikörper und Derivate davon gemäss Anspruch 4, die von einer Hybridomazellinie gemäss einem der Ansprüche 1 bis 3 produziert werden.

7. Monoklonaler Antikörper und Derivate davon gemäss Anspruch 4, der von einer Hybridomazellinie produziert wird, welche die kennzeichnenden Charakteristika von ECACC 9002 1701 besitzt.

8. Verfahren zur Herstellung einer Hybridomazellinie gemäss Anspruch 1, dadurch gekennzeichnet, dass man
a) ein Metolachlorderivat synthetisiert und mit einem Carriermolekül konjugiert
b) ein Donor-Tier mit besagtem Konjugat immunisiert
c) eine immunkompetente B-Zelle aus dem immunisierten Donor-Tier isoliert
d) besagte immunkompetente B-Zelle mit einer zur kontinuierlichen Zellteilung befähigten Tumorzellinie fusioniert.
e) das entstehende Fusionsprodukt isoliert, in einem geeigneten Kulturmedium kultiviert und anschliessend positive Hybridzellen kloniert und
f) die klonierten Hybridzellen auf die Bildung monoklonaler Antikörper hin untersucht und diejenigen selektioniert, welche mit den in Tabelle 1 aufgeführten Metolachlorderivaten < 2% kreuzreagieren.

9. Verfahren gemäss Anspruch 8, dadurch gekennzeichnet, dass es sich bei besagtem Metolachlorderivat um eine Verbindung der Formel (I) handelt, 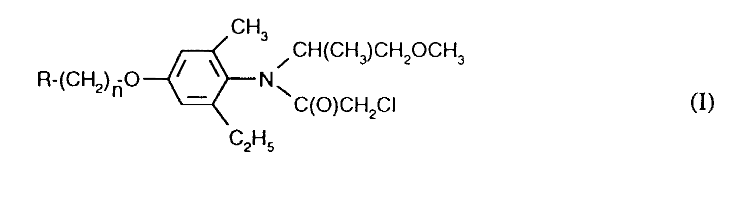 welche in 4 Stellung zur Aminofunktion des Metolachlor eine R-(CH₂)ₙ-O-Gruppierung aufweist, worin
R für COOH, NH₂ oder SH steht und
n eine ganze Zahl von 1 bis 10 repräsentiert.

10. Verfahren gemäss Anspruch 8, dadurch gekennzeichnet, dass man als Carriermolekül makromolekulare Verbindungen verwendet, die frei zugängliche reaktive Gruppen für die Kupplungsreaktion mit einem geeigneten Metolachlorderivat aufweisen und die in der Lage sind besagtem Metolachlor eine immunogene Potenz zu verleihen.

11. Verfahren gemäss Anspruch 10, dadurch gekennzeichnet, dass man ein lysinreiches Protein mit einem Molekulargewicht zwischen 10'000 und 1'500'000 verwendet.

12. Verfahren gemäss Anspruch 10, dadurch gekennzeichnet, dass man ein Protein ausgewählt aus der Gruppe bestehend aus "Bovine Serum Albumin" (BSA), "Keyhole Limpet Hemocyanin" (KLH), Humanes Serumalbumin (HSA), "Porcine Thyroglobulin", B2 Microglobulin, Hemocyanin, Immunoglobuline; Toxine; Polysaccharide; Lipopolysaccharide; natürliche oder synthetische Polyadenyl- und Polyuridylsäuren; Polyalanyl- und Polylysin-Polypeptide; oder Zellmembrankomponenten verwendet.

13. Verfahren gemäss Anspruch 8, dadurch gekennzeichnet, dass die Konjugation des Metolachlor an das Carriermolekül direkt erfolgt.

14. Verfahren gemäss Anspruch 8, dadurch gekennzeichnet, dass die Konjugation des Metolachlor an das Carriermolekül über ein Brückenglied (Spacer) erfolgt.

15. Verfahren gemäss Anspruch 14, dadurch gekennzeichnet, dass besagtes Brückenglied eine oder mehrere reaktive Gruppen besitzt, die in der Lage sind mit den reaktiven Gruppen des Carriermoleküls in Wechselwirkung zu treten.

16. Verfahren gemäss Anspruch 15, dadurch gekennzeichnet, dass es sich bei besagten reaktiven Gruppen um Carboxyl-, Amino- oder SH-Gruppen handelt.

17. Verfahren gemäss Anspruch 8, dadurch gekennzeichnet, dass die Kupplungsreaktion mit Hilfe der aktiven Estermethode durchgeführt wird.

18. Verfahren gemäss Anspruch 8, dadurch gekennzeichnet, dass die Immunisierung der Donor-Tiere durch ein- bis mehrmalige Applikation von Carrier-gebundenem Metolachlor erfolgt.

19. Verfahren gemäss Anspruch 18, dadurch gekennzeichnet, dass die Applikation in Form einer intravenösen, intra peritonealen oder subcutanen Injektion oder einer Kombination davon erfolgt.

20. Verfahren gemäss Anspruch 8, dadurch gekennzeichnet, dass man für die Fusion immunkompetenter Zellen aus dem Donor-Tier Tumorzellinien verwendet, die selbst keine monoklonalen Antikörper produzieren.

21. Verfahren gemäss Anspruch 20, dadurch gekennzeichnet, dass man Myelomazellinien verwendet, die die kennzeichnenden Charakteristika von Sp2/O-Ag14 oder X63-Ag8.653 besitzen.

22. Verfahren gemäss Anspruch 8, dadurch gekennzeichnet, dass man als Fusionsmedium eine Pufferlösung verwendet, die eines der üblicherweise für die Fusionierung von Zellen verwendeten Fusionspromotoren enthält ausgewählt aus der Gruppe bestehend aus: Sendai Viren oder andere Paramyxoviren, Calcium-Ionen, oberflächenaktive Lipide oder Polyethylenglykol.

23. Verfahren gemäss Anspruch 22, dadurch gekennzeichnet, dass es sich bei besagten Fusionspromotoren um Polyethylenglykol mit einem mittleren Molekulargewicht von 600 bis 6000 handelt.

24. Verfahren gemäss Anspruch 23, dadurch gekennzeichnet, dass die Polyethylenglykolkonzentration im Fusionssmedium 30 % - 60 % beträgt.

25. Verfahren gemäss Anspruch 8, dadurch gekennzeichnet, dass man für die Selektion fusionierte Hybridzellen das HAT-Selektionsmedium verwendet.

26. Verfahren gemäss Anspruch 8, dadurch gekennzeichnet, dass man die Hybridzellen in Gegenwart von isolierten Makrophagen ("Feederzellen") kultiviert.

27. Verfahren gemäss Anspruch 8, dadurch gekennzeichnet, dass man positive, monoklonale Antikörper produzierende Hybridzellkulturen mit Hilfe des limitierenden Verdünnungsverfahrens vereinzelt und anschliessend in geeigneten Kultivierungsmedien kloniert.

28. Verfahren gemäss Anspruch 8, dadurch gekennzeichnet, dass man die gemäss Anspruch 8 klonierten Hybridoma- Zellklone mit Hilfe eines Immunoassays auf die Bildung geeigneter monoklonaler Antikörper hin untersucht.

29. Verfahren gemäss Anspruch 28, dadurch gekennzeichnet, dass man einen Enzym-gekoppelten Immunassay oder einen Radioimmunassay verwendet.

30. Verfahren zur Herstellung monoklonaler Antikörper, dadurch gekennzeichnet, dass man die gemäss einem der Ansprüche 8 bis 28 hergestellten Hybridomazellinien *in vivo* oder *in vitro* mit Hilfe bekannter Verfahren kultiviert und die produzierten monoklonalen Antikörper isoliert.

31. Verfahren gemäss Anspruch 30, dadurch dadurch gekennzeichnet, dass man eine in vitro-Kultivierung in geeigneten Kultivierungsmedien durchführt.

32. Verfahren gemäss Anspruch 31, dadurch gekennzeichnet, dass man standardisierte Kulturmedien verwendet ausgewählt aus der Gruppe bestehend aus "Dulbecco's Modified Eagle Medium" (DMEM) oder RPMI 1640, die gegebenenfalls durch Zugabe von Säuger-Seren, durch wachstumsfördernde Zusätze oder durch Spurenelemente ergänzt werden können.

33. Verfahren gemäss Anspruch 30, dadurch gekennzeichnet, dass man besagte monoklonale Antikörper durch Expansion der gemäss einem der Ansprüche 8 bis 29 hergestellten Hybridomazellinien in einem Donor-Tier *in vivo* produziert.

34. Verfahren zum immunologischen Nachweis von Metolachlor, dadurch gekennzeichnet, dass man einen monoklonalen Antikörper der Ansprüche 4 bis 7 in einem der bekannten Immunassays verwendet.

35. Verfahren gemäss Anspruch 34, dadurch gekennzeichnet, dass es sich um einen kompetitiven Immunassay handelt.

36. Verfahren gemäss Anspruch 34, dadurch gekennzeichnet, dass es sich bei besagtem Immunassay um einen Radioimmunassay (RIA), einen enzymgekoppelten Assay (ELISA) oder einen Chemilumineszenzassay handelt.

37. Verfahren gemäss Anspruch 36, dadruch gekennzeichnet, dass es sich bei besagtem enzymgekoppelten Assay um einen indirekten ELISA handelt.

38. Verfahren gemäss Anspruch 36, dadruch gekennzeichnet, dass es sich bei besagtem enzymgekoppelten Assay um einen direkten ELISA handelt.

39. Mittel zum immunologischen Nachweis von Metolachlor in Form eines gebrauchsfertigen Test-Kits, dadurch gekenn zeichnet, dass besagter Test-Kit neben den üblicherweise verwendeten Trägermaterialien, Reagentien und sonstigen Zusätzen mindestens einen monoklonalen Antikörper gemäss einem der Ansprüche 4 bis 7 als Reagenz enthält.

40. Metolachlorderivat der Formel (I) zur Verwendung in einem Verfahren gemäss Anspruch 8 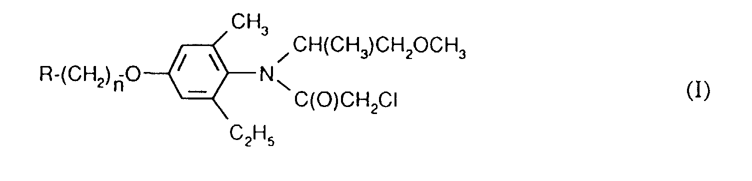 zur Kupplung an ein Trägermolekül, welches in 4 Stellung zur Aminofunktion des Metolachlor eine R-(CH₂)ₙ-O- Gruppierung aufweist, worin
R für COOH, NH₂ oder SH steht und
n eine ganze Zahl von 1 bis 10 repräsentiert.

41. Verfahren zur Herstellung eines zur Kupplung an ein Trägermolekül befähigten Metolachlorderivats der Formel I 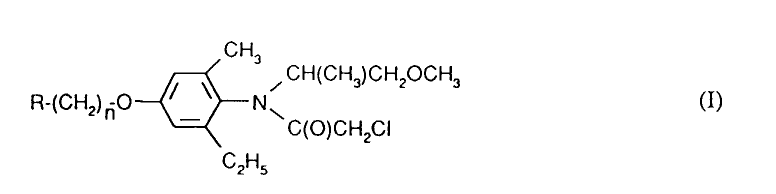 zur Verwendung in einem Verfahren gemäss Anspruch 8, welche in 4 Stellung zur Aminofunktion des Metolachlor eine R-(CH₂)ₙ-O- Gruppierung aufweist, worin
R für COOH, NH₂ oder SH steht und
n eine ganze Zahl von 1 bis 10 repräsentiert,
dadurch gekennzeichnet, dass man eine Verbindung der Formel II 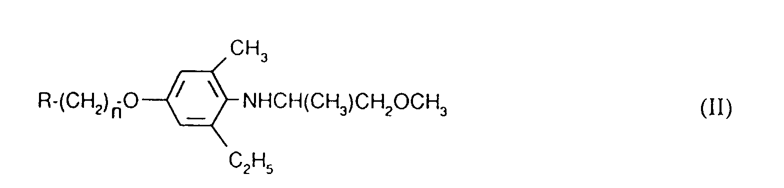 worin R und n die unter Formel I angegebenen Bedeutungen haben, in einem reaktionsinerten Lösungsmittel, unter milden Bedingungen bei einer Temperatur von -10 C bis + 30 C mit einem zur N-Acylierung befähigten Chloressigsäurederivat N-acyliert.

## Claims

1. A hybridoma cell line which produces a monoclonal antibody that is specific for metolachlor, characterised in that the cross-reactivity of the antibody with the structurally related metolachlor analogues indicated in Table 1 is < 2%.

2. A hybridoma cell line according to claim 1, which produces a monoclonal antibody having the cross-reactivity pattern reproduced in Table 1.

3. A hybridoma cell line according to claim 1, which has the characterising features of ECACC 9002 1701.

4. A monoclonal antibody that is specific for metolachlor and derivatives thereof, which have cross-reactivity with the structurally related metolachlor analogues indicated in Table 1 of < 2%.

5. Monoclonal antibody and derivatives thereof according to claim 4, which have the cross-reactivity pattern reproduced in Table 1.

6. Monoclonal antibody and derivatives thereof according to claim 4, which are prepared from a hybridoma cell line according to one of claims 1 to 3.

7. Monoclonal antibody and derivatives thereof according to claim 4, which is prepared from a hybridoma cell line having the characterising features of ECACC 9002 1701.

8. Process for the production of a hybridoma cell line according to claim 1, characterised in that
a) a metolachlor derivative is synthesised and conjugated with a carrier molecule
b) a donor animal is immunised with said conjugate
c) an immunologically competent B-cell from the immunised donor animal is isolated
d) said immunologically competent B-cell is fused with a tumour cell line capable of continuous cell division
e) the resulting fusion product is isolated, cultivated in an appropriate culture medium and thereafter positive hybrid cells are cloned, and
f) the cloned hybrid cells are examined for the formation of monoclonal antibodies, and those which cross-react by < 2% with the metalochlor derivatives listed in Table 1 are selected.

9. Process according to claim 8, characterised in that the metolachlor derivative in question is a compound of formula (I) 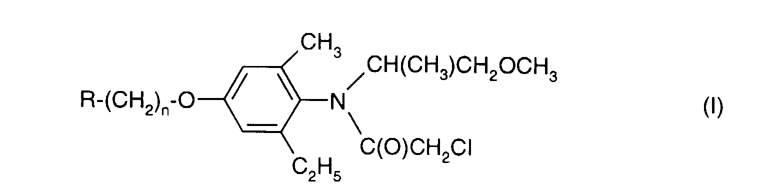 which has a R-(CH₂)ₙ-O- grouping in position 4 to the amino function, wherein
R is COOH, NH₂ or SH and
n represents a whole number from 1 to 10.

10. Process according to claim 8, characterised in that macromolecular compounds are used as the carrier molecule, and these have freely accessible reactive groups for the coupling reaction with an appropriate metolachlor derivative and are in a position to impart immunogenic power to said metolachlor.

11. Process according to claim 10, characterised in that a lysine-rich protein with a molecular weight of between 10,000 and 1,500,000 is used.

12. Process according to claim 10, characterised in that the protein employed is selected from the group comprising bovine serum albumin (BSA), keyhole limpet haemocyanin (KLH), human serum albumin (HSA), porcine thyroglobulin, B2 microglobulin, haemocyanin, immunoglobulins; toxins; polysaccharides; lipopolysaccharides; natural or synthetic polyadenylic and polyuridylic acids; polyalanyl- and polylysine-polypeptides; or cell membrane components.

13. Process according to claim 8, characterised in that conjugation of the metolachlor takes place directly on the carrier molecule.

14. Process according to claim 8, characterised in that conjugation of the metolachlor takes place on the carrier molecule via a bridging member (spacer).

15. Process according to claim 14, characterised in that said bridging member has one or more reactive groups which are in a position to interact with the reactive groups of the carrier molecule.

16. Process according to claim 15, characterised in that the reactive groups in question are carboxyl, amino or SH groups.

17. Process according to claim 8, characterised in that the coupling reaction is carried out by means of the active ester method.

18. Process according to claim 8, characterised in that immunisation of the donor animals is effected by application once or more of carrier-bound metolachlor.

19. Process according to claim 18, characterised in that application is effected in the form of an intravenous, intraperitoneal or subcutaneous injection or a combination thereof.

20. Process according to claim 8, characterised in that fusion of immunologically competent cells from the donor animal is carried out using tumour cell lines which themselves do not produce monoclonal antibodies.

21. Process according to claim 20, characterised in that myeloma cell lines are used, which have the characterising features of Sp2/O-Ag14 or X63-Ag8.653.

22. Process according to claim 8, characterised in that the fusion medium used is a buffer solution, which contains one of the fusion promotors customarily employed for the fusioning of cells, and selected from the group comprising: Sendai viruses or other paramyxo viruses, calcium ions, surface-active lipids or polyethylene glycol.

23. Process according to claim 22, characterised in that the fusion promotor in question is polyethylene glycol with an average molecular weight of 600 to 6000.

24. Process according to claim 23, characterised in that the polyethylene glycol concentration in the fusion medium is 30 % - 60 %.

25. Process according to claim 8, characterised in that the HAT selection medium is used for the selection of fusioned hybrid cells.

26. Process according to claim 8, characterised in that the hybrid cells are cultivated in the presence of isolated macrophages (feeder cells).

27. Process according to claim 8, characterised in that positive hybrid cell cultures producing monoclonal antibodies are isolated by means of the limiting dilution process and then cloned in appropriate cultivating media.

28. Process according to claim 8, characterised in that the hybridoma cell clone cloned in accordance with claim 8 is examined by immunoassay for the formation of suitable monoclonal antibodies.

29. Process according to claim 28, characterised in that the immunoassay may be enzyme-linked or may be a radioactive immunoassay.

30. Process for the production of monoclonal antibodies, characterised in that the hybridoma cell lines produced according to one of claims 8 to 28 are cultivated *in vivo* or *in vitro* by means of known processes, and the monoclonal antibodies produced are isolated.

31. Process according to claim 30, characterised in that an *in vitro* cultivation is carried out in suitable cultivation media.

32. Process according to claim 31, characterised in that standardised culture media are used, which are selected from the group comprising Dulbecco's Modified Eagle Medium (DMEM) or RPMI 1640, which may be optionally supplemented by adding mammals' serum, by growth-enhancing additives or by trace elements.

33. Process according to claim 30, characterised in that said monoclonal antibodies are produced *in vivo* in a donor animal by expansion of the hybridoma cell lines produced according to one of claims 8 to 29.

34. Process for the immunological detection of metolachlor, characterised in that a monoclonal antibody of claims 4 to 7 is used in one of the known immunoassays.

35. Process according to claim 34, characterised in that the immunoassay in question is a competitive immunoassay.

36. Process according to claim 34, characterised in that said immunoassay is a radioimmunoassay (RIA), an enzyme-linked assay (ELISA) or a chemiluminescence assay.

37. Process according to claim 36, characterised in that said enzyme-linked assay is an indirect ELISA.

38. Process according to claim 36, characterised in that said enzyme-linked assay is a direct ELISA.

39. Means for the immunological detection of metolachlor in the form of a ready-to-use test kit, characterised in that, in addition to the carrier materials, reagents and other additives normally used, said test kit also contains as reagent at least one monoclonal antibody according to one of claims 4 to 7.

40. Metolachlor derivative of formula (I) for use in a process according to claim 8 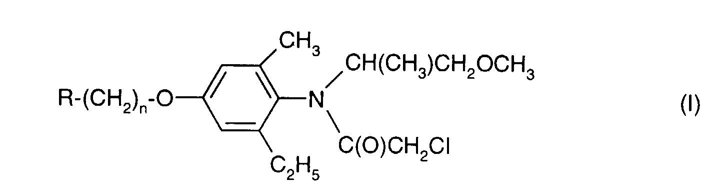 for coupling with a carrier molecule, which has a R-(CH₂)ₙ-O- grouping in position 4 to the amino function of the metolachlor, wherein
R is COOH, NH₂ or SH, and
n represents a whole number from 1 to 10.

41. Process for the production of a metolachlor derivative of formula I 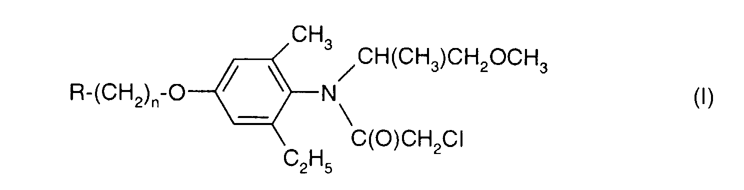 which is capable of coupling with a carrier molecule, for use in a process according to claim 8, which has a R-(CH₂)ₙ-O- grouping in position 4 to the amino function of the metolachlor, wherein
R is COOH, NH₂ or SH, and
n represents a whole number from 1 to 10,
characterised in that a compound of formula II 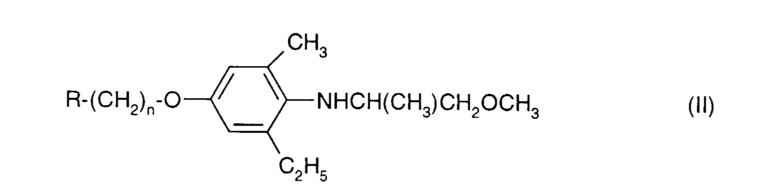 wherein R and n have the significances indicated under formula I,
is N-acylated with a chloroacetic acid derivative capable of N-acylation, in a solvent which is inert towards the reaction, under mild conditions, at a temperature of -10°C to +30°C.

## Revendications

1. Une lignée cellulaire d'hybridomes qui produit un anticorps monoclonal spécifique pour le Métolachlore, caractérisée en ce que les réactivités croisées de l'anticorps avec les analogues du Métolachlore apparentés structurellement indiqués dans le tableau 1, sont < 2 %.

2. Une lignée cellulaire d'hybridomes selon la revendication 1, qui produit un anticorps monoclonal qui présente le modèle de réactivité croisée indiqué dans le tableau 1.

3. Une lignée cellulaire d'hybridomes selon la revendication 1, qui possède les caractéristiques spécifiques du ECACC 9002 1701.

4. Un anticorps monoclonal spécifique pour le Métolachlore et ses dérivés qui présente une réactivité croisée avec les analogues du Métolachlore structurellement apparentés indiqués dans le tableau 1, < 2 %.

5. Un anticorps monoclonal et ses dérivés selon la revendication 4, qui présente un modèle de réactivité croisée indiqué dans le tableau 1.

6. Un anticorps monoclonal et ses dérivés selon la revendication 4, qui peut être produit à partir d'une lignée cellulaire d'hybridomes selon l'une des revendications 1 à 3.

7. Un anticorps monoclonal et ses dérivés selon la revendication 4, qui peut être produit à partir d'une lignée cellulaire d'hybridomes qui possède les caractéristiques spécifiques du ECACC 9002 1701.

8. Un procédé de préparation d'une lignée cellulaire d'hybridomes selon la revendication 1, caractérisé en ce que
a) on synthétise un dérivé du Métolachlore et on le conjugue avec une molécule porteuse,
b) on immunise un animal donneur avec le conjugué indiqué,
c) on isole une cellule B immunocompétente de l'animal donneur immunisé,
d) on fusionne ladite cellule B immunocompétente avec une lignée cellulaire tumorale capable de division cellulaire continue,
e) on isole le produit de fusion obtenu, on le cultive dans un milieu de culture approprié et on clone ensuite les cellules hybrides positives, et
f) on vérifie les cellules hybrides clonées afin de détecter la formation d'anticorps monoclonaux et on sélectionne celles dont la réaction croisée avec les dérivés du Métolachlore indiqués dans le tableau 1, est < 2 %.

9. Un procédé selon la revendication 8, caractérisé en ce que ledit dérivé du Métolachlore est un composé de formule (I) 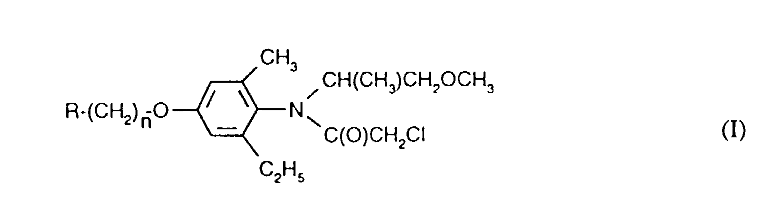 qui, en position 4 de la fonction amino du Métolachlore, possède un groupement R-(CH₂)ₙ-O-, où
R signifie COOH, NH₂ ou SH, et
n signifie un nombre entier de 1 à 10.

10. Un procédé selon la revendication 8, caractérisé en ce qu'on utilise comme molécule porteuse des composés macromoléculaires qui présentent des groupes réactifs facilement accessibles pour la réaction de couplage avec un dérivé approprié du Métolachlore et qui sont capables de conférer une puissance immunogène audit Métolachlore.

11. Un procédé selon la revendication 10, caractérisé en ce qu'on utilise une protéine riche en lysine ayant un poids moléculaire compris entre 10 000 et 1 500 000.

12. Un procédé selon la revendication 10, caractérisé en ce qu'on choisit une protéine parmi le groupe comprenant "la sérum-albumine bovine" (SAB), "l'hémocyanine de patelle" (KLH), "la sérum-albumine humaine" (SAH), "la thyroglobuline Porcine", la microglobuline B2, l'hémocyanine, l'immunoglobuline; les toxines; les polysaccharides; les lipopolysaccharides; les acides polyadényliques et polyuridyliques naturels ou synthétiques; les polyalanyl- et polylysin-polypeptides; ou bien les composants de la membrane cellulaire....

13. Un procédé selon la revendication 8, caractérisé en ce que la conjugation du Métolachlore à la molécule porteuse s'effectue directement.

14. Un procédé selon la revendication 8, caractérisé en ce que la conjugation du Métolachlore à la molécule porteuse s'effectue via un membre formant pont (espaceur).

15. Un procédé selon la revendication 14, caractérisé en ce que ledit membre formant pont possède un ou plusieurs groupes réactifs qui sont capables d'entrer en interaction avec les groupes réactifs de la molécule porteuse.

16. Un procédé selon la revendication 15, caractérisé en ce que lesdits groupes réactifs sont les groupes carboxyle, amino ou SH.

17. Un procédé selon la revendication 8, caractérisé en ce que la réaction de couplage est effectuée à l'aide de la méthode de l'ester actif.

18. Un procédé selon la revendication 8, caractérisé en ce que l'immunisation des animaux donneurs est effectuée par application unique ou répétée de Métolachlore lié au porteur.

19. Un procédé selon la revendication 18, caractérisé en ce que l'application est effectuée par injection intraveineuse, intra-péritonéale ou sous-cutanée ou par une de leurs combinaisons.

20. Un procédé selon la revendication 8, caractérisé en ce que pour la fusion on utilise des cellules immunocompétentes issues de lignées cellulaires tumorales des animaux donneurs qui elles-mêmes ne produisent pas d'anticorps monoclonaux.

21. Un procédé selon la revendication 20, caractérisé en ce qu'on utilise des lignées cellulaires de myélomes qui ont les caractéristiques spécifiques du Sp2/O-Ag14 ou du X63-Ag8.653.

22. Un procédé selon la revendication 8, caractérisé en ce que comme milieu de fusion, on utilise une solution tampon qui contient un des promoteurs de fusion habituels pour la fusion des cellules qui est choisi parmi le groupe comprenant: les virus Sendai ou d'autres paramyxovirus, les ions calcium, les lipides actifs en surface ou le polyéthylèneglycol.

23. Un procédé selon la revendication 22, caractérisé en ce que lesdits promoteurs de fusion sont le polyéthylèneglycol d'un poids moléculaire moyen de 600 à 6000.

24. Un procédé selon la revendication 23, caractérisé en ce que la concentration en polyéthylèneglycol dans le milieu de fusion est de 30 à 60 %.

25. Un procédé selon la revendication 8, caractérisé en ce que pour la sélection des cellules hybrides fusionnées on utilise le milieu de sélection HAT.

26. Un procédé selon la revendication 8, caractérisé en ce qu'on cultive les cellules hybrides en présence de macrophages isolés ("cellules Feeder").

27. Un procédé selon la revendication 8, caractérisé en ce qu'on sépare des cultures de cellules hybrides positives produisant des anticorps monoclonaux à l'aide du procédé de dilution limitée et on les clone ensuite dans des milieux de culture appropriés.

28. Un procédé selon la revendication 8, caractérisé en ce qu'on vérifie les clones cellulaires d'hybridomes clonés selon la revendication 8, à l'aide d'un essai immunologique afin de détecter la formation d'anticorps monoclonaux appropriés.

29. Un procédé selon la revendication 28, caractérisé en ce qu'on utilise un essai immunologique couplé à une enzyme ou une méthode de dosage radioimmunologique.

30. Un procédé de préparation d'anticorps monoclonaux, caractérisé en ce qu'on cultive in vivo ou in vitro les lignées cellulaires d'hybridomes préparées selon l'une des revendications 8 à 28 à l'aide d'un procédé connu et on isole les anticorps monoclonaux produits.

31. Un procédé selon la revendication 30, caractérisé en ce qu'on effectue une culture in vitro dans des milieux de culture appropriés.

32. Un procédé selon la revendication 31, caractérisé en ce qu'on utilise des milieux de culture standardisés choisis parmi le groupe comprenant le "Milieu d'Eagle modifié par Dulbecco" (DMEM) ou le RPMI 1640, qui peuvent éventuellement être complétés par addition de sérum de mammifères, par des additifs favorisant la croissance ou par des éléments traceurs.

33. Un procédé selon la revendication 30, caractérisé en ce qu'on produit lesdits anticorps monoclonaux in vivo dans un animal donneur, par expansion des lignées cellulaires d'hybridomes préparées selon l'une des revendications 8 à 29.

34. Un procédé de détection immunologique du Métolachlore, caractérisé en ce qu'on utilise un anticorps monoclonal selon les revendications 4 à 7 dans un des essais immunologiques connus.

35. Un procédé selon la revendication 34, caractérisé en ce qu'il s'agit d'un essai immunologique compétitif.

36. Un procédé selon la revendication 34, caractérisé en ce que ledit essai immunologique est une méthode de dosage radioimmunologique (RIA), un essai couplé à l'enzyme (ELISA) ou un essai de chimioluminescence.

37. Un procédé selon la revendication 36, caractérisé en ce que ledit essai couplé à l'enzyme est un essai ELISA indirect.

38. Un procédé selon la revendication 36, caractérisé en ce que ledit essai couplé à l'enzyme est un essai ELISA direct.

39. Un agent de détection immunologique du Métolachlore sous forme d'un kit d'essai prêt à l'emploi, caractérisé en ce que ledit kit d'essai, outre les matières porteuses habituelles, les réactifs et les autres additifs, contient au moins un anticorps monoclonal selon l'une des revendications 4 à 7 comme réactif.

40. Un dérivé du Métolachlore de formule (I) pour une utilisation dans un procédé selon la revendication 8 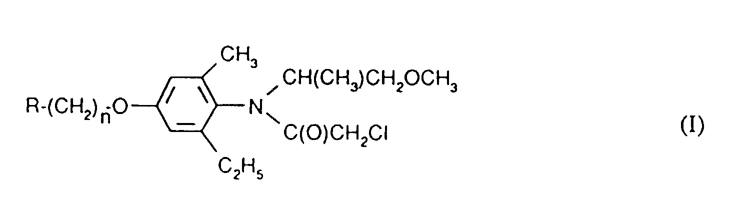 pour le couplage à une molécule porteuse, qui, en position 4 de la fonction amino du Métolachlore, présente un groupement R-(CH₂)ₙ-O-, où
R signifie COOH, NH₂ ou SH, et
n signifie un nombre entier de 1 à 10.

41. Un procédé de préparation d'un dérivé du Métolachlore pour le couplage à une molécule porteuse de formule I 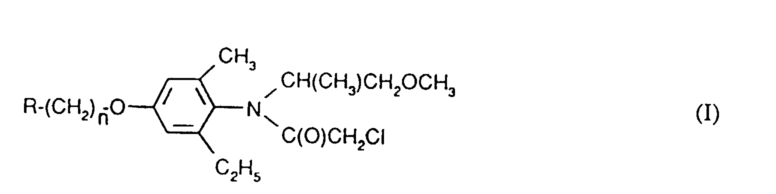 pour l'utilisation dans un procédé selon la revendication 8, qui en position 4 de la fonction amino du Métolachlore présente un groupement R-(CH₂)ₙ-O-, où
R signifie COOH, NH₂ ou SH, et
n signifie un nombre entier de 1 à 10,
caractérisé en ce qu'on N-acyle un composé de formule II 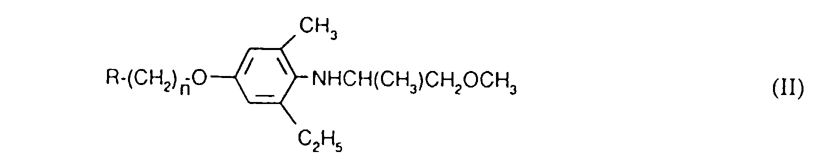 où R et n ont les significations indiquées sous la formule I, avec un dérivé de l'acide chloracétique approprié pour la N-acylation, dans un solvant qui est inerte à la réaction, sous des conditions douces, à une température de -10°C à + 30°C.
